# EUROPEAN PATENT SPECIFICATION

(11) **EP 0 676 952 B1**
(45) Date of publication and mention of the grant of the patent: **12.07.2000**
(21) Application number: 94903641.2
(22) Date of filing: 15.12.1993
(51) Int. Cl.: A61K 9/08

(54) **BIOERODIBLE CONTROLLED DELIVERY SYSTEM**
BIOERODIERBARES SYSTEM ZUR KONTROLLIERTEN FREIGABE
SYSTEME DE LIBERATION CONTROLEE BIOERODABLE

(30) Priority: 29.12.1992 US 997913
(43) Date of publication of application: 18.10.1995
(73) Proprietor: INSITE VISION INCORPORATED, Alameda, CA 94501 (US)
(72) Inventor: BOWMAN, Lyle M., Pleasanton, CA 94566 (US); CHANDRASEKARAN, Santosh Kumar, Morgana, CA 94556 (US); PATEL, Rajesh, San Mateo, CA 94404 (US); VO, Hoa Vinh, Alameda, CA 94501 (US)
(74) Representative: Goldin, Douglas Michael
(86) International application number: US9312170
(87) International publication number: WO9414416

(56) References cited:
- WO-A-92/11843
- FR-A- 2 299 356
- US-A- 4 115 544

## Description

The present invention is concerned with sustained release delivery systems for medicaments, especially topical ophthalmic delivery systems. More particularly, the present invention relates to a two-phase, sustained release drug delivery system in which a bioerodible polymeric matrix material is dispersed or suspended as a discontinuous phase in a continuous phase hydrophobic liquid material.

In topical administration of medicaments to the eye, a variety of factors can be important, among them; comfort, consistency and accuracy of dosage, type and time of any vision interference, ease of administration, and timing of delivery. Prior ophthalmic delivery vehicles have suffered drawbacks in one or more of those areas.

For example, eyedrops in the form of aqueous solutions or suspensions can be rapidly washed away by the eye's tear fluid. Ointments or creams can blur the vision and oftentimes can result in other undesirable effects. Gelatin lamellae or other films or sheets, ocular inserts and non-aqueous suspensions and emulsions all can cause immediate pain and continuing discomfort and can also interfere with vision. A number of drug delivery systems or formulations have been developed in an attempt to ameliorate or to avoid the foregoing problems.

One group of polymers found useful in the eye are disclosed in Choi et al, U.S. Patent No. 4,138,344, issued February 6, 1979. In particular, Choi et al disclose orthoester and orthocarbonate polymers having a repeating mer comprising a hydrocarbon radical and a symmetrical dioxycarbon unit. The polymers are generally highly viscous or solid and have been prepared for use in the form of a bioerodible insert encapsulated by another polymer.

The bioerodible ocular insert of Choi et al consists of a bioerodible polymer comprising a continuous matrix in which particles of drug are dispersed. When the ocular insert is placed in the environment of the eye, the polymer gradually bioerodes and releases drug to the eye and surrounding tissues. Until now, it is believed that the polymers have been suitable for administration to the eye only as an insert because of their tackiness and poor handling ability.

US 4,115,544 discloses an ophthalmic dosage in the form of solid particles that can be suspended in a liquid medium at a time prior to use, said particles comprising ophthalmic drug dispersed throughout a bioerodible drug release rate controlling material.

The present invention evolved from efforts to obtain sustained release benefits from such polymers without constraints and disadvantages associated with inserts.

It is an object of the present invention to provide novel sustained release delivery systems.

It is a particular object of this invention to provide novel sustained release delivery systems for topical ophthalmic delivery of medicaments.

A further object of this invention is to provide novel, sustained release, topical ophthalmic delivery systems suitable for administration of medicaments at intervals of once daily or even longer.

Yet another object of this invention is to provide a method for convenient therapeutic treatment using a delivery system which has a prolonged release time for medicaments.

A still further object of this invention is to provide novel processes for the preparation of sustained release delivery systems.

Thus, the invention provides a sustained release topical ophthalmic medicament delivery system for administration to the eye, comprising:
a hydrophobic liquid component in a continuous phase;
an essentially bioerodible component which is comprised of a bioerodible polymer and which forms a matrix material for carrying medicament, the bioerodible polymer being a polymer which breaks down by chemical decomposition upon reaction with biological materials;
the essentially bioerodible component being dispersed or suspended as a discontinuous phase in said hydrophobic liquid component;
the relative amounts of said hydrophobic liquid component and said bioerodible component being such that the viscosity of the system is in the range of 1-100 Pa.s and suitable for administration to the eye in ribbon or drop form;
medicament carried in part by the bioerodible component and also carried by or entrapped directly in the continuous phase; and
   wherein the essentially bioerodible component acts to hold the formulation, including the medicament, together and, in the topical ophthalmic application, to increase residence time.

The invention also provides a sustained release medicament delivery system, comprising:
a hydrophobic liquid component in a continuous phase; and
an essentially bioerodible component which is comprised of a bioerodible polymer which breaks down by chemical decomposition upon reaction with biological materials;
the essentially bioerodible component being dispersed or suspended as a discontinuous phase in said hydrophobic liquid component;
medicament carried in part by said bioerodible component in said discontinuous phase and also carried by or entrapped directly in the continuous phase; and
   wherein the essentially bioerodible component acts to hold the formulation, including the medicament, together.

The invention further provides a sustained release medicament delivery system, for administration to the eye, comprising:
a continuous phase liquid component selected from the group consisting of mineral oil and silicone oil;
a bioerodible component selected from the group consisting of plasticized and unplasticized polyorthoesters;
the relative amounts of said liquid component and said bioerodible component being such that the viscosity of the system is in the range of 5-100 Pa.s. and suitable for administration to the eye in ribbon or drop form;
said bioerodible component being dispersed or suspended as a discontinuous phase in said liquid component;
medicament carried in part by the bioerodible component and also carried by or entrapped directly in the continuous phase; and
   wherein the essentially bioerodible component acts to hold the formulation, including the medicament, together and, in topical ophthalmic applications, to increase residence time.

In accordance with a preferred form of the invention intended to accomplish at least some of the foregoing objects, a sustained release medicament delivery system comprises a hydrophobic liquid component in a continuous phase and an essentially bioerodible component which is comprised of a bioerodible polymer. The essentially bioerodible component is dispersed or suspended as a discontinuous phase in the hydrophobic liquid component. Medicament is carried in part by the bioerodible component in the discontinuous phase and in part by the continuous phase itself. Preferably, the bioerodible polymer forms a matrix material for carrying much if not most of the medicament.

When formulated as a topical ophthalmic delivery system, the viscosity of the composition is desirably in a range suitable for administration to the eye in ribbon form or in drop form. Preferably that viscosity is from about 1 to about 55 Pa.s. For drops the viscosity is more preferably from about 5 to about 30 Pa.s., and for administration in ribbon form the viscosity is more preferably from about 40 to about 55 Pa.s.. There can, however, be overlap in the drop and ribbon viscosity ranges by reason of variations in formulation techniques and ingredients. When formulated for injection, them viscosity of the injectable liquid can be substantially greater than 100 Pa.s., but still preferably such that the liquid can be passed through an 18 gauge or smaller needle at room temperature (about 20°C). Oral formulations as viscous liquids are also contemplated.

The essentially bioerodible component comprises (and preferably primarily comprises, and more preferably consists essentially of) a plasticized or unplasticized bioerodible polymer. As used herein "erodible" and "bioerodible" refer to the property of the polymer to break down as a unit structure by chemical decomposition, as opposed to physical degradation, e.g., by the polymer reacting with water or through polymer reaction with enzymes, water in tear fluids or other biological materials.

Preferred plasticizers are, polyethylene glycols, glycerin, propylene glycol, polypropylene glycol, ethylene glycol, cetyl alcohol and polyvinyl alcohol. In topical ophthalmic preparations only small amounts of ethylene glycol should be used because of potential toxicity in the eye.

When included, the plasticizers are present in any suitable amount which does not preclude either bioerodibility or the formation of the discontinuous phase. Generally, however, plasticizers when employed will be present in an amount up to about 40% of the total weight of the essentially bioerodible component preferably about 5% to about 30%, more preferably to about 10% to about 25%. In any event, the amount of plasticizer or any other constituent in the discontinuous phase material is such that the component remains essentially bioerodible.

Plasticized polymer delivery systems useful in the present invention as the essentially bioerodible component to be dispersed as a discontinuous phase in the hydrophobic liquid component are disclosed in a related application WO9414417 entitled "Plasticized Bioerodible Controlled Delivery System",

The bioerodible polymeric material of the discontinuous phase is preferably selected from the group consisting of polysacchrides, proteinaceous polymers and their soluble derivatives, polypeptides, polyesters, polylactic acid polymers, polyglycolic acid polymers, poly(lactic/glycolic) copolymers and polyorthoesters. The most preferred materials are plasticized and unplasticized polyorthoesters, especially poly(2,2 dioxo-trans-1,4 cyclohexane dimethylene tetrahydrofuran), of the type disclosed by Choi et al, U.S. Patent No. 4,128,344

The hydrophobic liquid material of the continuous phase is preferably mineral oil or silicone oil. However other oils, including vegetable oils, are also contemplated.

The viscosity of hydrophobic liquid material selected can be within a wide range, for example from about 1 to about 55 Pa.s. The bioerodible polymeric material of the discontinuous phase will generally contribute to an even higher system viscosity. Accordingly, when the viscosity of the selected hydrophobic liquid material of the continuous phase is itself relatively high as compared with other choices of hydrophobic liquid materials, less bioerodible polymeric material will normally be used to maintain the system viscosity in the desired range. However, if more bioerodible polymer is desired, perhaps to increase the overall medicament delivery time, a lower viscosity hydrophobic liquid material may be selected.

The essentially bioerodible component is preferably present in the delivery system in an amount within a range of about 10% to about 60%, more preferably about 20% to about 50%, by weight based on the weight of the entire delivery system (excluding medicament). The hydrophobic liquid material component is preferably present in an amount within the range of about 90% to about 40%, more preferably about 80% to about 50%, by weight of the entire delivery system (excluding medicament). When amounts of hydrophobic liquid material at the higher end of the range are employed, there sometimes can be a tendency toward an undesired type of phase separation which impedes the suspension or dispersion of the essentially bioerodible component, in which case a reduction in the proportion of the hydrophobic liquid material may be desirable.

Where topical ophthalmic compositions useful to ameliorate "dry eye" conditions are formulated, the medicament, as that term is here used, may be a demulcent. Of course, the term medicament also includes, and in most instances will be constituted by, what are often referred to as over the counter or prescription drugs. Drugs administered by means of the controlled release drug delivery systems of the present invention preferably include an adrenocorticotrophic hormone, insulin, vitamin, steroid, narcotic antagonist, antibiotic, anticancer drug, antihypertensive drug, aminosteriod, polypeptides or protein. The drugs may be in either the free base or the acid form. Particular advantages are perceived for administering water soluble drugs inasmuch as they are believed less likely to diffuse out of the system. Therefore, their administration is more erosion controlled.

According to some method aspects of the present invention, a process for making a sustained release medicament system comprises the steps of suspending or dispersing an essentially bioerodible component, which comprises (and preferably primarily comprises, and more preferably consists essentially of) an essentially bioerodible polymer, as a discontinuous phase in a continuous phase of a hydrophobic liquid component, and carrying medicament in the two-phase system. At least some of the medicament is carried in the discontinuous phase by the bioerodible component. In this connection, although the present invention provides for entrapping (i.e. dispersing or dissolving) at least some medicament in the discontinuous phase, a viable bioerodible delivery system is envisioned even when medicament is largely carried by or entrapped directly in the continuous phase. In such cases, the essentially bioerodible polymer component acts to hold the formulation, including the medicament, together and, in topical opthalmic application, increases residence time.

Depending on its molecular weight, the bioerodible polymer employed can be at room temperature a tacky, solid or semi-solid substance, or a tacky, extremely viscous liquid. In order to enhance the flowability of the system and/or the homogeneity of the suspension or dispersion, the essentially bioerodible component is rendered more flowable by heating, plasticizing, or both. Preferably, the more flowable essentially bioerodible component and the hydrophobic liquid component are actively mixed, as by stirring, at an elevated temperature. Plasticizing can reduce or minimize the need for heat that might adversely affect the stability of the medicament. Plasticizing also reduces the tackiness of the polymer.

Another method aspect of the present invention involves suspending or dispersing the essentially bioerodible component as a discontinuous phase in a continuous phase of a hydrophobic liquid component, heating the essentially bioerodible component for a time sufficient to substantially reduce its molecular weight, and cooling the bioerodible component to room temperature. It has been found particularly desirable with higher molecular weight forms of the preferred poly(2,2-dioxo-trans-1,4-cyclohexane tetrahydrofuran) polymer to heat the polymer at an elevated temperature for a time sufficient to substantially reduce its molecular weight. This aids in preventing the system from solidifying at room temperature, especially when the bioerodible polymer constitutes 50% or more of the composition. Heating to a temperature range of about 14°C to about 180°C for 30 minutes to ten days is recommended to advantageously reduce the polymer molecular weight. Preferably at least some of the heating occurs in the presence of the hydrophobic liquid component and during active mixing, as by stirring, to further enhance the homogeneity of the suspension or dispersion. The system is then cooled to room temperature. Alternatively, a low molecular weight polymer may be used.

In order to minimize the potential for irritation (e.g. eye irritation) as a result of byproducts produced during molecular weight reduction (e.g. decomposition products such as gamma-butyrolactone and cyclohexanyldimethanol, when the preferred polyorthoesters are employed), it can be advantageous to pull a vacuum during heating to remove such byproducts, especially those byproducts whose vapor pressure is sufficiently high to be amenable to removal as gas.

The entrapping of at least some of the medicament in the discontinuous phase can be accomplished in a variety of ways. One way is to mix the medicament with the bioerodible polymer, in a flowable condition, prior to suspending or dispersing the essentially bioerodible component in the hydrophobic liquid component. The bioerodible polymer may be placed in that flowable condition by heating, plasticizing or both.

While such direct mixing of the medicament with the bioerodible polymer might enhance the amount of medicament carried in the discontinuous phase, it is generally preferred to add the medicament together with the bioerodible polymer and the hydrophobic liquid component. The timing, however, of that addition should be determined with consideration given to the heat sensitivity of the medicament, which might be unstable at some elevated temperatures, or when heated for longer times.

In such circumstances, it can be advantageous to first heat and actively mix the bioerodible polymer and the hydrophobic liquid, and then to add the medicament, preferably again with active mixing, either during cooldown or during a reheat step under less severe temperature or time conditions. Since the intended phase separation, which is fully developed at room temperature (to yield the suspension or dispersion of the essentially bioerodible component as a discontinuous phase in the hydrophobic liquid component), is not fully developed during such cooldown or has been reversed in whole or in part during such reheat, the entrapment of the medicament in the discontinuous phase can be enhanced.

When medicament is added to the combination of the bioerodible polymer and the hydrophobic liquid, it is envisioned that at least some of the medicament will also be entrapped directly in the continuous phase. However, even when that is not the case, the essentially bioerodible component in the discontinuous phase appears to act as a sort of binder which enhances the integrity of the system as a whole, thereby also enhancing the controlled release even of medicament which is carried directly by the continuous phase hydrophobic liquid component.

It is also possible to prepare the formulation by dissolving the bioerodible polymer and the medicament in a suitable solvent for both of them. The solvent is then stripped off in any suitable manner (for example, by heating or pulling a vacuum), leaving the medicament suspended in the polymer to which the hydrophobic liquid is added. The solvent should be non-toxic to the desired target tissue and easily removeable. It should also be essentially free of water. A suitable solvent for poly (2,2 dioxo-trans-1,4 cyclohexane dimethylene tetrahydrofuran) is ethanol. Other suitable solvents such as other alcoholic solvents, tetrahydrofuran, methylene chloride or the like may desirably be employed.

In preparing some "dry eye" formulations according to the present invention, separate demulcents may be added as medicament. However, it is also contemplated that some plasticizers, such as polyethylene glycol, employed to reduce viscosity of the essentially bioerodible component can themselves constitute medicament, by acting as a lubricant carried by the bioerodible component.

In accordance with further aspects of the present invention there is thus provided a therapeutic treatment which has sustained integrity of the delivery system and a prolonged release time for medicament. A multi-phase delivery system is formulated with the essentially bioerodible component dispersed or suspended as a discontinuous phase in a continuous phase hydrophobic liquid component. Medicament is carried in the system, in part by the discontinuous phase. The system carrying the medicament is delivered to a mammalian organism in need of treatment by the medicament, and medicament release is controlled by bioerosion of the discontinuous phase.

In a topical opthalmic treatment, the viscosity of the system is adjusted so as to be suitable for administration to the eye in ribbon or drop form, and controlled release of medicament to the eye preferably can take place over a prolonged time, even a period of twenty-four hours or longer. The system may also be formulated as a liquid which is injected into the mammalian organism through an 18 guage or smaller needle.

Other objects, advantages and aspects of the present invention will become apparent from the detailed description below.

A wide variety of bioerodible polymers are contemplated as useful in preparing controlled drug delivery systems according to the present invention. The below mentioned bioerodible polymers are believed particularly suited for the methods and compositions of the present invention by reason of their characteristically low human toxicity and virtually complete biodegradability. Of course, it will be understood that any bioerodible polymer may be useful in the practice of the present invention in its broadest form as long as it is non-toxic and can be degraded by chemical decomposition through contact with body fluids. Further, different molecular weights of polymers may be used in the practice of the present invention as long as the appropriate controlled release feature of the formulation as a result of bioerosion is maintained.

Polymers useful in the present invention may be derived from a variety of sources and should be formulated to be or behave as a bioerodible polymer in the sense that they biodegrade by reaction with water, enzymes or other biological materials encountered upon administration to the body of a mammalian organism. Such polymers include polysaccharides, proteinaceous polymers and their soluble derivatives, polypeptides, polyesters, polylactic acid polymers, polyglycolic acid polymers, poly(lactide/glycolide) copolymers, polyorthoesters, and the like.

The polysaccharides may be poly-1,4-glucans, e.g., starch glycogen, amylose and amylopectin, and the like. Preferably, such a bioerodible polymer is a water-soluble derivative of a poly-1,4-glucan, including hydrolyzed amylopectin, hydroxyalkyl derivatives of hydrolyzed amylopectin such as hydroxyethyl starch, hydroxyethyl amylose, dialdehyde starch, and the like.

Proteinaceous polymers and their soluble derivatives include gelatin, biodegradable synthetic polypeptides, elastin, alkylated collagen, alkylated elastin, and the like.

Biodegradable synthetic polypeptides include poly-(N-hydroxyalkyl)-L-asparagine, poly-(N-hydroxyalkyl)-L-glutamine, copolymers of N-hydroxyalkyl-L-asparagine and N-hydroxyalkyl-L-glutamine with other amino acids. Suggested amino acids include L-alanine, L-lysine, L-phenylalanine, L-leucine, L-valine, L-tyrosine, and the like.

Definitions or further descriptions of any of the foregoing polymers are well known in the art and may be found by referring to any standard biochemistry reference text such as "Biochemistry" by Albert L. Lehninger, Worth Publishers, Inc. and "Biochemistry" by Lubert Stryer, W.H. Freeman and Company,

Polyorthoesters are preferred in the practice of the present invention. Those polymers are normally a highly viscous, tacky substance at low molecular weight, and a rigid, tacky solid at high molecular weight. Choi et al, U.S. Patent No. 4,138,344 issued February 6, 1979, describes polyorthoesters comprising hydrocarbon radicals and a symmetrical dioxycarbon unit with a multiplicity of organic groups bonded thereto. In particular, Choi, et al discloses polymers comprising a carbon-oxygen backbone having a dioxycarbon moiety with a plurality of organic groups pendant from the dioxycarbon. The polymers are represented by the following general formula: 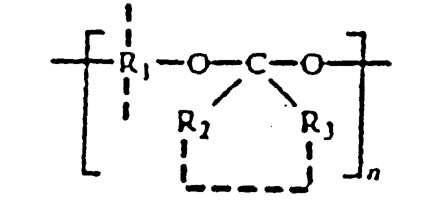 wherein R₁ is a di, tri or tetravalent alkylene, alkenylene, alkyleneoxy, cycloalkylene, cycloalkylene substituted with an alkyl, alkoxy or alkenyl, cycloalkenylene, cycloalkenylene substituted with an alkyl, alkoxy, alkenyloxy, alkylene, alkenylene, alkyleneoxy, alkenylencoxy, alkylenedioxy, alkenylenedioxy, aryloxy, aralkyleneoxy, aralkenyleneoxy, aralkylene dioxy, aralkenylenedioxy, oxa, or OR₁O with R₁ defined as above; and wherein, a) R₁ is divalent when R₂ and R₃ are alkyl, alkenyl, alkoxy, or alkenyloxy, with at least one of R₂ or R₃ an alkoxy or alkenyloxy; b) R₁ is divalent when R₂ and R₃ are intramolecularly covalently bonded to each other and to the same dioxycarbon atom to form a heterocyclic ring or a heterocyclic ring substituted with an alkyl, alkoxy or alkenyl when R₂ is an alkyleneoxy or alkenyleneoxy and R₃ is an alkyleneoxy, alkenyleneoxy or alkylene; c) R₁ is divalent when R₂ and R₃ are intramolecularly covalently bonded to each other and to the same dioxy carbon atom to form a fused polycyclic ring or a fused polycyclic ring substituted with an alkyl, alkoxy or alkenyl when R₂ is an oxa, alkyleneoxy or alkenyleneoxy and R₃ is aryloxy, aralkyleneoxy, aralkenyleneoxy or aralkylene; d) R₁ is divalent when R₂ or R₃ is an OR₁O bridge between polymer backbones bonded through their dioxycarbon moieties, and the other R₂ or R₃ is an alkyl, alkenyl, alkyloxy, or alkenyloxy e) R₁ is tri or tetravalent when R₂ and R₃ are covalently bonded to each other and to the same dioxycarbon atom to form a heterocyclic ring or a heterocyclic ring substituted with an alkyl, alkoxy or alkenyl when R₂ is an alkyleneoxy or alkenyleneoxy and R₃ is an alkyleneoxy, or alkylene; and f) R₁ is tri or tetravalent when R₂ and R₃ are covalently bonded to each other and to the same dioxy carbon atom to form a fused polycyclic ring or fused polycyclic ring substituted with an alkyl, alkoxy or alkenyl when R₂ is an oxa, alkyleneoxy or alkenyleneoxy and R₃ is aryloxy, aralkyleneoxy, aralkenyleneoxy or aralkylene.

The polymers contemplated as useful in the practice of the present invention include homopolymers, copolymers of the random and block types formed by reacting monomers or mixtures of preformed homopolymers and/or copolymers, branched polymers and cross-linked polymers. In addition, thermoplastic linear polymers when R₁ is divalent, R₂ and R₃ are substituted with a non-crosslinking group or are bonded intramolecularly, thermosetting cross-linked polymers when R₁ is divalent and R₂ or R₃ is intermolecularly bonded between different polymeric backbones; and, thermosetting cross-linked polymers when R₁ is tri or tetravalent and R₂ and R₃ are substituted with non-crosslinking groups, or bonded intramolecularly, are useful.

The phrase hydrocarbon radical appearing above and as used elsewhere in the specification, includes, for the purpose of this invention, the terms embraced by R₁, R₂ and R₃ as defined below.

The term alkylene used in this specification for R₁ denotes a straight or branched chain divalent, trivalent or tetravalent alkylene radical of 2 to 10 carbon atoms inclusive such as 1,2-ethylene; 1,3-propylene; 1,2-propylene; 1,4-butylene; 1,5-pentylene; 1,6-hexylene; 1,2,5-hexylene; 1,3,6-hexylene; 1,7-heptylene; 2-methyl-1,7-heptylene; 1,8-octylene; 1,10-decylene; 2-propyl-1,6-hexylene; 1,1-dimethyl-1,6-hexylene; and the like. These alkylene chains are derived from the corresponding glycols.

The term alkenylene used for R₁ denotes an unsaturated straight or branched chain multivalent radical having 2 to 10 carbon atoms such as 1,4-but-2-enylene; 1,6-hex-3-enylene; 1,7-hept-3-enylene; 1,8-oct-3-enylene; 1,9-non-3-enylene; 4-propyl-(1,6-hex-3-enylene); 5-methoxy-(1,6-hex-3-enylene); 2-propenyl-(1,6-hex-3-enylene); and the like.

The term cycloalkylene as used for R₁ includes monocyclic, lower cycloalkylene radicals of 3 to 7 carbons such as cyclopropylene; cyclobutylene; cyclopentylene; cyclohexylene and cycloheptylene. Similarly, the phrase cycloalkylene substituted with an alkyl of 1 to 7 carbons, an alkoxy of 1 to 7 carbons, or an alkenyl of 2 to 7 carbons, includes substituted cycloalkylenes such as 2-methyl-1,3-cyclopropylene; 2-methyl-1,4-cyclopentylene; 2-methyl-1,6-cyclohexylene; 2-ethoxy-2,3-cyclopentylene; 2-methyl-1,6-cyclohexylene; 2-ethoxy-2,3-cyclopropylene; 5-butoxy-1,4-cyclopentylene; 2-methoxy-1,4-cyclohexylene; 2-propenyl-1,5-cyclopentylene; 2-isobutenyl-1,6-cyclohexylene; and the like.

Exemplary R₁ cycloalkenylene and R₁ cycloalkenylene substituted with an alkyl of 1 to 7 carbons, an alkoxy of 1 to 7 carbons, or an alkenyl of 2 to 7 carbons, include monocyclic alkylenes having from 4 to 7 carbons as ring members, such as 1,4-cyclopent-2-enylene; 1,5-cyclopent-3-enylene; 1,6-cyclohex-2-enylene; 1,6-cyclohex-2-enylene; and the substituted rings such as 5-methyl-(1,4-cyclopent-2-enylene); 6-ethyl-(1,4-cyclohex-2-enylene); 6-ethoxy-(1,5-cyclohex-2-enylene); 2-propyl-(1,5-cyclohex-3-enylene); 2-methoxy-(1,4-cyclohex-2-enylene); 2-methoxy-(1,4-cyclohept-2-enylene), and the like.

The expressions R₁ arylene and R₁ arylene substituted with an alkyl of 1 to 7 carbons an alkenyl of 2 to 7 carbons, or an alkoxy of 1 to 7 carbons, include the benzenoid groups such as phenylene, phenylalkylene and phenylalkenylene. Typical groups are 1,4-phenylene; 1,4-phenyldimethylene; 1,4-phenyldiethylene; 2,ethyl-1,4-phenyldimethylene; 2-methyl-1,4-phenyldimethylene; 2-methoxy-(1,4-phenyldimethylene); 2-propyl-(1,4-phenyldiethylene); and the like.

The term alkyl appearing herein for R₂ and R₃, and as a substituent on the aryl, cycloalkyl and heterocyclic group, embraces straight and branched chain alkyl radicals of 1 to 7 carbon atoms such as methyl; ethyl; n-propyl; n-butyl, n-amyl, n-hexyl; n-heptyl and the various positional isomers thereof such as isopropyl; t-butyl; sec-butyl; isoamyl; isohexyl; t-heptyl and the like.

Exemplary alkenyls as used for R₂ and R₃, and as a substituent on the aryl, cycloalkyl and heterocyclic group, include the straight and branched chain lower alkenyl groups of 2 to 7 carbons such as 1-propenyl; 2-propenyl or allyl; 1-butenyl; 2-butenyl; 1-pentenyl; 2-ethenyl; and the corresponding positional isomers such as 1-isobutenyl; 2-isobutenyl; 2-sec-butenyl; 2-methyl-1-butenyl; 2-methyl-2-pentenyl- 2,3-dimethyl-3-hexenyl and the like.

The term alkoxy as used for R₂ and R₃, and as a substituent on the aryl, cycloalkyl and heterocyclic group, include the straight and branched chain lower alkoxy groups and the positional isomers thereof having 1 to 7 carbon atoms inclusive, for example, methoxy; ethoxy; propoxy; butoxy; n-pentoxy; n-hexoxy isopropoxy; 2-butoxy; isobutoxy; 3-pentoxy; and the like.

The term alkenyloxy as used for R₂ and R₃ embraces the straight and branched chain lower alkenyloxy groups and the positional isomers thereof having 2 to 7 carbon atoms, for example, ethenoxy; propenoxy; butenoxy; pentenoxy; hexenoxy; isopropenoxy; isobutenoxy; sec-butenoxy; 2-methyl-1-butenoxy; 2-methyl-2-butenoxy; 2,3-dimethyl-3-butenoxy; and the like.

The term alkylenoxy appearing in the general formula comprehends, for R₁, R₂ and R₃, straight and branched chain alkylenoxy radicals of the formula ―OR₄― wherein R₄ is an alkylene of 2 to 6 carbons, for example, 1,3-propyleneoxy; 1,4-butyleneoxy; 1,5-pentyleneoxy and the like. Similarly, the term alkenyleneoxy comprehends, for R₂ and R₃, radicals of the general formula ―OR₅― wherein R₅ is an alkenylene of 3 to 6 carbons, such as prop-1-enyleneoxy; 1,4-but-1-enyleneoxy; 1,4-but-2-enyleneoxy; 1,5-pent-1-enyleneoxy; 1,5-hex-1-enyleneoxy and the like.

The expressions alkylenedioxy and alkenyldioxy include the straight and branched chain radicals of the formula ―OR₄O― wherein R₄ is an alkylene of 2 to 6 carbons and of the formula ―OR₅O― wherein R₅ is an alkenylene of 3 to 6 carbons, such as for alkylenedioxy propylenedioxy; butylenedioxy; pentylenedioxy; hexylenedioxy; and heptylenedioxy and for alkenylenedioxy; prop-1-enylenedioxy 1,4-but-1-enylenedioxy 1,4-but-2-enylenedioxy; 1,5-pent-1-enylenedioxy; 1,6-hex-1-enylenedioxy; and 1,7-hep-1-enylenedioxy. The phrase heterocyclic ring of 5 to 8 carbons for R₂ and R₃, defines the ring formed when R₂ or R₃ is a bond, alkylene or alkenylene, and at least one of R₂ or R₃ is an alkyleneoxy, alkenyleneoxy, alkylenedioxy or alkenylenedioxy with the terms as defined above.

The terms alkylene and alkenylene used when R₂ and R₃ are independently taken together to form a ring in cooperation with the carbon of the carbox-oxygen polymeric backbone, include an alkylene of 2 to 6 carbons and an alkylene of 3 to 6 carbons, such as the alkylenes: 1,2-ethylene, 1,3-propylene, 1,4-butylene, 1,5-pentylene, and 1,6-hexylene, and the alkenylenes: 1,3-prop-1-enylene, 1,4-but-1-enylene, 1,4-but-2-enylene, 1,5-pent-1-enylene, 1,6-hex-2-enylene, and 1,7-hept-2-enylene.

The terms aryloxy, aralkyleneoxy, aralkenyleneoxy, aralkylenedioxy and aralkenylenedioxy used for R₂ and R₃ include a radical of 8 to 12 carbons wherein aryloxy is ar-O-, alkyleneoxy is ―OR₄―, alkenyleneoxy is ―OR₅―, alkylenedioxy is ―OR₄O―, alkenylenedioxy is ―OR₅O―, with R₄ is alkylene and R₅ an alkenylene as defined above, and ar is phenyl. The phrase fused polycyclic ring of 8 to 12 carbons as used herein, defines a substituent in which a heterocyclic and an aryl ring have two atoms in common, for example, benzfuryl; benzpyranyl; 4,5-benz-1,3-dioxepanyl; 5,6-benz-1,3-dioxepanyl; 4-5-benz-1,3-dioxolanyl; 4,5-benz-1,3-dioxyolanyl; 4,5-benz-1,3-dioxocanyl; 5,6-benz-1,3-dioxocanyl; 6,7-benz-1,3-dioxocanyl; 7,8-benz-1,3-dioxocanyl, and benz-1,3-dioxoanyl.

The term mer as used herein for polymers, copolymers and terpolymers denotes the member unit or the monomeric unit of the polymer. For example, in a homopolymer, the mer units are the same. In a copolymer, there are at least two different mer units. They can be ordered in the polymer chain in a random fashion when the original units are copolymerized in a common reaction vessel, or they can be ordered in block fashion when the polymers are combined after an initial homopolymerization of each of the different monomeric units. A terpolymer is a copolymer with at least a third mer unit.

The preferred polyorthoester for use in the practice of the present invention is poly(2,2-dioxo-trans-1,4-cyclohexane dimethylene tetrahydrofuran).

The polymer erodes by reacting with water to form non-toxic degradation products. The polymer responds to attack by water and degrades by reaction at a rate which depends on the final formulation. The primary degradation products of poly (2,2 dioxo-trans-1,4 cyclohexane dimethylene tetrahydrofuran) are dimethanol cyclohexane and gamma-butyrolactone. Degradation is retarded by base and accelerated by acid.

Advantageously, the polymer system has the ability to deliver medicament at a relatively constant rate over a prolonged period of time. The degradation rate may vary from approximately several hours to several months depending on the formulation parameters as well as the physical properties of the polymer selected. The degradation of the polymer occurs when the dispersion or suspension is administered to a mammalian organism and is exposed to water. With polyorthoesters such as poly(2,2-dioxo-trans-1,4-cyclohexene dimethylene tetrahydrofuran) formulations according to the present invention, it is contemplated that topical opthalmic delivery by a once-a-day eye drop may be achievable.

The amount of polymer appropriate to achieve a desired controlled release effect from bioerosion will vary from formulation to formulation. The amount of polymer should not, however, be so large as to place the final formulation in solid form, taking into account molecular weight reductions achievable by heating or by polymer synthesis, and also taking into account contributions to flowability by addition of plasticizers. Where molecular weight reductions are undertaken, generally reductions to a molecular weight of about 2,000 to about 4,000 are believed appropriate.

A liquid delivery system wherein an essentially bioerodible component is dispersed or suspended as discontinuous phase in a continuous phase hydrophobic liquid component characterizes the formulations of the present invention. A component is essentially bioerodible when formulated such that it mainly degrades by reaction with water, enzymes or other biological materials encountered upon administration to the body of a mammalian organism. The amount of the essentially bioerodible component, which primarily comprises and preferably consists essentially of the bioerodible polymer, plasticized or unplasticized, generally represents about 10 to about 60% by weight of the entire controlled release formulation, preferably about 20 to about 50%.

The polymer of the present invention, whether present merely as a dispersion or as a suspension (i.e. a basically uniform dispersion), is carried by the hydrophobic liquid material. The polymer exists in a discontinuous phase, such as balls, droplets, particles or microparticles separated from one another, within the hydrophobic liquid continuous phase.

Suitable hydrophobic liquid materials used to provide the continuous phase in which the bioerodible phase is dispersed according to the present invention are hydrophobic liquid materials identified in a variety of standard reference textbooks including The Merck Index and Reminginton's Pharmaceutical Sciences Silicone oil and mineral oil are preferred. However, other oils are also contemplated, such as: olive oil and fatty oils of vegetable origin, e.g., corn oil (maize oil), cottonseed oil, peanut oil and sesame oil. While the bioerodible polymer typically exists in a solid state, the hydrophobic liquid material disperses or suspends the polymer thereby allowing the polymer to be formulated for administration in a more compliant composition which retains advantageous controlled release properties of the polymer. The hydrophobic liquid material selected also should not react with the polymer or the medicament, and should be safe for administration to a mammalian organism. Different hydrophobic liquid materials may be more suitable for different routes of administration, so that different hydrophobic liquid materials may be selected for oral administration, topical ophthalmic administration, or parenteral administration. However, the preferred materials, silicone oil and mineral oil are believed suitable for all of those routes.

The amount of hydrophobic liquid material which is present in the controlled release formulations of the present invention is, as stated above, sufficient to disperse or suspend the bioerodible component as a discontinuous phase while also retaining controlled release advantages of the bioerodible polymer. The amount of the hydrophobic liquid material generally represents about 90 to about 40% by weight of the entire controlled release drug delivery system, and preferably about 80 to about 50% by weight.

Formulations of the present invention should have a viscosity that is suited for the selected route of administration. Generally, the viscosity ranges for topical opthalmic administration will be from about 1 to about 100 Pa.s.. Approximately 5-30 Pa.s. is an advantageous viscosity range for ophthalmic administration in drop form. For administration in ribbon form, the viscosity is preferably from about 4 to about 100 Pa.s. For parenteral administration the formulation preferably has a viscosity suitable for injection through an 18 guage, or smaller, needle at room temperature (about 20°C).

When packaged for delivery after a longer shelf life or storage time, standard or other suitable preservatives may be added to the formulations.

The controlled release systems of the present invention are intended to release a drug or other medicament. Medicaments -- substances used in treating or ameliorating a disease or medical condition -- including drugs intended to therapeutically treat a mammalian organism, e.g., human, will typically be incorporated in the controlled release drug delivery system of this invention in therapeutically active amounts comparable to amounts administered in other dosage forms, usually, in amounts ranging from about 0.005% to about 20% by weight, and preferably from about 0.01% to about 20% by weight, based on the total weight of the formulation. Thus, for example, from about 0.01% to about 1% by weight of the anti-inflammatory steroid fluorometholone can be administered in this manner.

An illustrative but by no means exhaustive listing of such medicaments includes demulcents (for relief of "dry eye"), vasoconstrictors, antibiotics, antivirals, steroids, amino-substituted steroids, steroidal and non-steroidal anti-inflammatory agents, peptides, polypeptides, cardiotonics, antihypertensives, antiallergics, alpha- and beta-adrenergic blocking agents, ophthalmic medicaments such as anticataract agents, antiglaucoma agents and ophthalmic antiinflammatory agents, ophthalmic lubricating agents, ophthalmic topical or regional anesthetic agents, and the like.

Specific medicaments believed suitable for use in the present invention include drugs such as idoxuridine, carbachol, bethanechol, timolol, atenolol, labetolol, metoprolol, nadolol, oxprenolol, pindolol, sotalol, betaxolol, acebutolol, alprenolol, levobunolol, p-aminoclonidine, dipivefrin, epinephrine, phenylephrine, phospholine, aceclidine, demecarium, cyclopentolate, homatropine, scopolamine, pilocarpine, ethacrynic acid, furosemide, amiloride, bacitracin, neomycin, polymyxin, polymyxin B, gramicidin, gentamycin, penicillins, erythromycin, sulfacetamide, tobramycin, trospectomycin, vancomycin, ciprofloxacin, perfloxacin, olfloxacin, enoxacin, naphazoline hydrochloride, clindamycin, isofluorophate, fluorometholone, dexamethasone, hydrocortisone, fluorocinolone, medrysone, methylprednisolone, fluticasone propionate, betamethasone, estradiol, ibuprofen, diclofenac, flurbiprofen, naproxen, esters of ibuprofen, flurbiprofen, naproxen, ketorolac, suprofen, interferons, cromolyn, gancyclovir, aminozolamide, all trans-retinoic acid (Vitamin A) and the nontoxic, pharmaceutically acceptable salts thereof. Pro-drug counterparts are also within the scope of the present invention.

Topical or regional anesthetic agents include ones used during ophthalmic surgery or other ophthalmic procedures, such as lidocaine, cocaine, benoxinate, dibucaine, proparacaine, tetracaine, etidocaine, procaine. hexylcaine, bupivacaine, mepivacaine, prilocaine, chloroprocaine, benzocaine, tetracaine, and the like, as well as their acid forms.

The term "drug" as comprehended by active agent, broadly includes physiologically or pharmacologically active substances for producing a localized or systemic effect or effects in mammals including humans and primates, avians, valuable domestic household, sport or farm animals such as sheep, goats, cattle, horses, etc., or for administering to laboratory animals such as mice, rats and guinea pigs. That is, the drug delivery system of the present invention can be used for administering drugs that are active at a point in near relation to the delivery system, or, for administering drugs which will produce a response at a site remote from the point of application of the drug delivery system. The drugs that may be administered include inorganic and organic drugs without limitation, are those drugs that can be transported across a vessel, for example, drugs acting on the central nervous system such as hypnotics and sedatives, mixtures thereof such as pentobarbital sodium, phenobarbital, secobarbital, thiopental, etc.; heterocyclic hypnotics such as dioxopiperidines, and glutarimides; hypnotics and sedatives such as amides and ureas exemplified by diethylisovaleramide and a bromo-isovaleryl urea; and hypnotic and sedative urethanes and disulfanes; narcotic antagonists such as naloxone and cyclazocine; psychic energizers such as isocarboxazid, nialamide, phenelzine, imipramine, tranylcypromine and paragylene; tranquilizers such as chloropromazine, promazine, fluphenazine, reserpine, deserpidine; meprobamate and benzodiazepines such as chlordiazepoxide; anticonvulsants such as primidone, diphenylhydantoin, ethyltoin, phenetruide and ethosuximide; muscle relaxants and anti-parkinson agents such as mephenesin, methocarbomal, trihexylphenidyl, biperiden and levo-dopa, also known as L-dopa and L-β-3-4dihydroxyphenylalanine; analgesics such as morphine, codeine, meperidine and nalorphine, antipyretics and anti-inflammatory agents such as aspirin, salicylamide and sodium salicylamide; local anesthetics such as procaine, lidocaine, naepaine, piperocaine, tetracaine, and dibucane; antispasmodics and antiulcer agents such as atrophic, scopolamine, methscopolamine, oxyphenonium, papaverine and prostaglandins such as PGE₁, PGE₂, PGF₁ₐ, PGF₂ₐ, and PGA; anti-microbials such as penicillin, tetracycline, oxytetracycline, chlortetracycline, and chloramphenicol; sulfonamides; anti-malarials such as 4-aminoquinolines, 8-aminoquinolines and pyrimethamine; antivirals including idoxuridine, hormonal agents such as prednisolone, prednisolone acetate, cortisone, cortisol and triamcinolone; androgenic steroids, for example methyltestosterone and fluoxmesterone; estrogenic steroids, for example, 17 β-estradiol and ethinyl estradiol; progestational steroids, for example, 17 α-hydroxyprogesterone acetate, 19-nor-progesterone, norethindrone and progesterone; sympathomimetic drugs such as epinephrine, amphetamine, ephedrine, and norepinephrine; cardiovascular drugs, for example, procainamide, amyl nitrite, nitroglycerin, dipyridamole, sodium nitrate, and mannitol nitrate; diuretics, for example, chlorothiazide, and flumethiazide; antiparasitic agents such as bephenium hydroxynaphthoate, dichlorophen, dapsone and enitabes; neoplastic agents such as mechlorethamine, uracil mustard, 5-fluorouracil, 6-thioguanine, and procarbazine; hypoglycemic drugs such as insulin, isophane insulin suspension, protamine zinc insulin suspension, globin zinc insulin, extended insulin zinc suspension, and other like insulins derived from animal and synthetic origin including tolbutamide, acetohexamide, tolazamide, and chlorpropamide; nutritional agents, for example vitamins such as ascorbic acid, essential amino acids, essential elements such as iron, and essential fats; ophthalmic drugs such as pilocarpine base, pilocarpine hydrochloride, pilocarpine nitrate, eserine, eserine salicylate, atropine sulfate, homatropine, and eucatropine, and proteins or peptides such as human epidermal growth factor (hEGF), aFGF, bFGF, IL-1ra, TGF-β and gamma-interferon. The above drugs are further described in "The Pharmacological Basis of Therapeutics," edited by Goodman and Gilman, published by The Macmillan Company.

Of the aforementioned drugs, adrenocorticotrophic hormone, insulin, vitamins, especially vitamins B₁₂, steroids, e.g., testosterone, progesterone and triancinolone, narcotic antagonists, e.g., naltrexone, cyclazocine and naloxone, antibiotics, e.g., tobramycin, anticancer drugs, e.g., cyclophosphamide, doxorubicin and cisplatin, antihypertensive drugs and proteins are especially contemplated in the practice of the present invention.

Another group of drugs are also especially contemplated in the practice of the present invention. Those drugs are the C₂₀ through C₂₆ amino substituted steroids of the following formula XI structure as set forth in WO 87/01706, especially those which exhibit antioxidant functions: 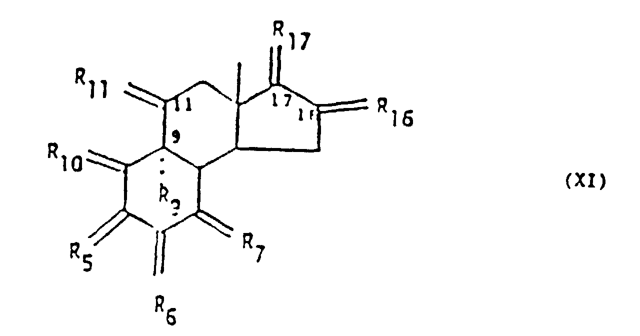 where:
(A-I) R₆ is α-R₆₁:β-R₆₂, R₁₀ is α-R₁₀₁:β-R₁₀₂ and R₇ is α-H:β-H, where one of R₆₁ and R₆₂ is -H, and the other is -H, -F, or C₁-C₃ alkyl, R₁₀₂ is -CH₃, R₁₀₁ and R₅ taken together are -(CH₂)₂-C(-R₃₃)-CH= or -CH-CH-CO-CH=, where R₃₃ is =O or α-H:β-OR₃₄ or α-OR₃₄:β-H, where R₃₄ is -H, -P(=O)(OH)₂, -CO-OH₃, -CO-C₂H₅, -CO-C₆H₅, -CO-O-CH₃ or -CO-O-C₂H₅;
(A-II) R₅ is α-R₅₃:β-R₅₄, R₆ is α-R₆₃:β-R₆₄, R₁₀ is α-R₁₀₃:β-R₁₀₄ and R₇ is α-H:β-H, where one of R₆₃ and R₆₄ is -H and the other taken together with one of R₅₃ and R₅₄ forms a second bond between C₅ and C₆, R₁₀₄ is -CH₃, R₁₀₃ and the other of R₅₃ and R₅₄ taken together are -(CH₂)₂-C(H)(OH)-CH₂-or -(CH₂)₂-C[H][OP(=O)-(OH)₂]-CH₂-;
(A-III) R₁₀ and R₅ taken together are =CH-CH=C(OR₃)-CH= where R₃ is -H, -P(-O)(OH)₂, C₁-C₃ alkyl, -CO-H, C₂-C₄ alkanoyl or benzyl, R₆ is α-R₆₅:β-R₆₆ where one of R₆₅ and R₆₆ is -H, and the other is -H, -F, or C₁-C₃ alkyl and R₇ is α-H:β-H;
(A-IV) R₅ is α-R₅₇:β-R₅₈, R₆ is α-R₆₇:β-R_{68,} R₇ is α-H:β-H and R₁₀ is α-R₁₀₇:β-R₁₀₈, where one of R₅₇ and R₅₈ is -H, R₁₀₇ and the other of R₅₇ and R₅₈ taken together are -(CH₂)₂-C(=R₃₃)-CH₂, where R₃₃ is as defined above, R₁₀₈ is -CH₃, where one of R₆₇ and R₆₈ is -H and the other is -H, -F, or C₁-C₃ alkyl;
(A-V) R₆ is R₆₉:R₆₁₀, R₇ is R₇₉:R₇₁₀, R₁₀ is α-R₁₀₉: R₁₀₁₀, where one of R₆₉ and R₆₁₀ is -H and the other taken together with one of R₇₉ and R₇₁₀ forms a second bond between C₆ and C₇, and the other of R₇₉ and R₇₁₀ is -H, R₁₀₁₀ is -CH₃, R₁₀₉ and R₅ taken together are -(CH₂)₂-C(=R₃₃)-CH= or -CH=CH-CO-CH=, where R₃₃ is as defined above; where:
(C-I) R₁₁ is α-R₁₁₁:β-R₁₁₂, where one of R₁₁₁ and R₁₁₂ is taken together with R₉ to form a second bond between C₉ and C₁₁ and the other of R₁₁₁ and R₁₁₂ is -H;
(C-II) R₉ is -Cl and R₁₁ is =O or α-H:β-R₁₁₄ where R₁₁₄ is -Cl or -OH;
(C-III) R₉ is -H or -F and R₁₁ is =O or α-R₁₁₅:β-R₁₁₆, where one of R₁₁₅ and R₁₁₆ is -H, and the other of R₁₁₅ and R₁₁₆ is -H, -OH or C₁-C₁₂ alkoxy;
(C-IV) R₉ is -H or -F and R₁₁ is α-O-CO-R₁₁₇:β-H, where R₁₁₇ is
   (A) C₁-C₃ alkyl,
   (B) C₁-C₁₂ alkoxy,
   (C) furanyl,
   (D) -NR₁₂₂R₁₂₃, where one of R₁₂₂ and R₁₂₃ is -H, methyl or ethyl and the other is -H, C₁-C₄ alkyl or phenyl,
   (E) -X₃-X₁, where X₃ is -O- or a valence bond, where X₁ is phenyl optionally substituted with 1 through 2 -Cl, -Br, C₁-C₃ alkoxy, -COOH, -NH₂, C₁-C₃ alkylamino, di(C₁-C₃)alkylamino, where the alkyl groups are the same or different, 1-pyrrolidinyl-, 1-piperidinyl, 1-hexamethylenimino-, 1-heptamethylenimino-, C₂-C₄ acylamino and -NH-CHO or with 1 -F or -CF₃; where:
(D-I) R₁₆ is R₁₆₁:R₁₆₂ and R₁₇ is R₁₇₁:R_{172,} where one of R₁₆₁ and R₁₆₂ is -H or -CH₃ and the other taken together with one of R₁₇₁ and R₁₇₂ forms a second bond between C₁₆ and C₁₇, and the other of R₁₇₁ and R₁₇₂ is -C(=Z)-(CH₂)ₙ-NR₂₁R₂₁₀, where Z is =O, =CH₂ or R₁₇₉:-H where R₁₇₉ is -H or -CH₃, where n is 0 through 6, where
   (A) R₂₁ is
      (1) -(CH₂)ₘ -NR₂₁₁-X₂, where m is 2, 3 or 4, where R₂₁₁ is -H or C₁-C₃ alkyl, where X₂ is: [A]
         (a) pyridin-2-, 3- or 4-yl or the N-oxide thereof optionally substituted by 1 or 2 R₂₁₂, being the same or different, where R₂₁₂ is
            (i) -F,
            (ii) -Cl,
            (iii) -Br,
            (iv) C₁-C₅ alkyl,
            (v) -CH₂-CH=CH₂,
            (vi) -X₁, where X₁ is as defined above,
            (vii) -NR₂₁₃R₂₁₃ where the R₂₁₃'s are the same or different and are -H, C₁-C₃ alkyl or -CH₂-CH=CH₂,
            (viiiα) *CH₂-(CH₂)_{q}-CH₂-N*- where the atoms marked with an asterisk (∗) are bonded to each other resulting in the formation of a ring, where q is 1 through 5,
            (viiiβ) *CH₂-CH₂-(CH₂)_{c}-G-(CH₂)_{d}-CH₂-CH₂-N*- where the atoms marked with an asterisk (∗) are bonded to each other resulting in the formation of a ring, where G is -O-, -S-, -SO-, -SO₂- or -NHR₂₁₄, where R₂₁₄ is -H, C₁-C₃ alkyl, or X₁ as defined above, where c and d are the same or different and are 0 through 2 with the proviso that the total number of ring carbon atoms is 4, 5 or 6, [a]
            (ix) 3-pyrrolin-1-yl, [b]
            (x) pyrrol-1-yl optionally substituted with C₁-C₃ alkyl, [c]
            (xi) piperidin-1-yl optionally substituted with 1 or 2 C₁-C₃ alkyl, [d]
            (xii) 1,2,3,6-tetrahydro-pyridin-1-yl, [e]
            (xiii) 1-hexamethyleneimino containing a 3-or 4-double bond or 3-and 5-double bonds, [f]
            (xiv) 1,4-dihydro-1-pyridinyl substituted in the 4 position by two C₁-C₃ alkyl being the same or different, [g]
            (xv) -OH,
            (xvi) C₁-C₃ alkoxy,
            (xvii) -NR₂₁₇-(CH₂)ₑ-Q where Q is 2-pyridinyl where R₂₁₇ is -H or C₁-C₃ alkyl and e is O through 3 [i]
            (xviii) pyridin-2-, 3- or 4-yl,
         (b) 1,3,5-triazin-4-yl or the N-oxide thereof optionally substituted at the 2- and/or 6- position with R₂₁₂ is as defined above, (4)
         (c) pyrimidin-4-yl or the N-oxide thereof optionally substituted at the 2- and/or 6- position with R₂₁₂ is as defined above, (5)
         (d) pyrimidin-2-yl optionally substituted at 4-and/or 6- position with 1 or 2 R₂₁₂ as is defined above, (6)
         (e) pyrazin-2-yl optionally substituted with 1 or 2 R₂₁₂ as is defined above, (7)
         (f) imidazol-2-yl optionally substituted in the 1 position with C₁-C₃ alkyl or -X₁, where X₁ is as defined above, and further optionally substituted with 1 or 2 R₂₁₂ as defined above, (8)
         (g) 1,3,4-triazol-2-yl optionally substituted in the 1 position with C₁-C₃ alkyl or -X₁, where X₁ is as defined above, and further optionally substituted with R₂₁₂ as defined above, (9)
         (h) imidazol-4- or 5-yl optionally substituted in the 1- position with C₁-C₃ alkyl or -X₁, where X₁ is as defined above, and further optionally substituted with 1 or 2 R₂₁₂ as defined above, (10)
         (i) benzo[b][thien-2-yl, (12a)
         (j) indol-2-yl, (12b)
         (k) benzo[b]thiazol-2-yl, (12c)
         (l) benzimidazol-2-yl, (12d)
         (m) 4-[2-[4-[2,6-bis(1-pyrrolidinyl)-4-pyrimidinyl]-1-piperazinyl]ethyl]-piperazinyl, (13)
         (n) 1,2,4-triazin-3-yl optionally substituted at the 5- and/or 6- position with R₂₁₂ as is defined above, (14)
      (2) (1-piperazinyl)-(C₂-C₄)alkyl optionally substituted in the 4- position with -X₁ or -X₂ as defined above, [B]
      (3) -X₂, as defined above, [O]
      (4) -(CH₂)ₘ-X₄ where in is as defined above and where X₄ is
         (a) -O-CH₂CH₂-Y, where Y is C₁-C₃ alkylamino, di(C₁-C₃)alkylamino where the alkyl groups are the same or different, C₃-C₆ alkyleneimino, optionally substituted with 1 or 2 C₁-C₃ alkyl,
         (b) -NR₂₂₀CH₂-CH₂-Y, where R₂₂₀ is -H or C₁-C₃ alkyl and Y is as defined above,
         (c) -(CH₂)_{g}-N(R₂₂₀)-X₂, where g is 2, 3 or 4, and where R₂₂₀ and X₂ are as defined above, [H]
      (5) -(CH₂)ₘ-NR₂₂₂R₂₂₃, where R₂₂₂ is -H or C₁-C₃ alkyl and R₂₂₃ is -X₁ or -X₂ as defined above, or R₂₂₂ and R₂₂₃ are taken together with the attached nitrogen atom to form a saturated mono-nitrogen C₃-C₆ heterocyclic ring and where m is as defined above, [I]
      (6) -(CHCH₃)_{b}-(CH₂)_{f}-R₂₂₄, where b is 0 and f is 1 through 3 or b is one and f is 0 through 3, where R₂₂₄ is phenyl substituted with 1 through 3 -OH, C₁-C₃ alkoxy, -NR₂₂₅R₂₂₆ where R₂₂₅ and R₂₂₆ are the same or different and are -H, C₁-C₃ alkyl or are taken together with the attached nitrogen atom to form a C₄-C₇ cyclicamino ring, [J]
      (7) -(CH₂)ᵢ-X₂, where i is 1 through 4 and X₂ is as defined above, [K]
      (8) (1-piperazinyl)acetyl substituted in the 4-position by X₂ where X₂ is as defined above, [L]
      (9) (1-piperazinyl)carbonylmethyl substituted in the 4-position by -X₂ where X₂ is as defined above, and [M]
   (B) R₂₁₀ is
      (1) -H,
      (2) C₁-C₃ alkyl,
      (3) C₅-C₇ cycloalkyl,
      (4) -(CH₂)ₘ-NR₂₁₁-X₂, where in, R₂₁₁ and X₂ are as defined above, [A]
      (5) (1-piperazinyl)-(C₂-C₄)alkyl optionally substituted in the 4- position with -X₁ or -X₂ as defined above, [B]
      (6) -(CH₂)ₘ-X₄, where m and X₄ are as defined above, [H]
      (7) -(CH₂)ₘ-NR₂₂₂R₂₂₃, where m, R₂₂₂ and R₂₂₃ are as defined above, [I]
      (8) -(CHCH₃)_{b}-(CH₂)_{f}-R₂₂₄, where b, f and R₂₂₄ are as defined above, [J]
   (C) R₂₁ and R₂₁₀ are taken together with the attached nitrogen atom to form a heterocyclic ring selected from the group consisting of
      (1) 2-(carboxy)-1-pyrrolidinyl optionally as the C₁-C₃ alkyl ester or as a pharmaceutically acceptable salt, [C-1]
      (2) 2-(carboxy)-1-piperidinyl optionally as the C₁-C₃ alkyl ester or as a pharmaceutically acceptable salt [C-2]
      (3) 2-(carboxy)-1-hexamethyleneimino optionally as the C₁-C₃ alkyl ester or as a pharmaceutically acceptable salt, [C-3]
      (4) 2-(carboxy)-1-heptamethylene-imino optionally as the C₁-C₃ alkyl ester or as a pharmaceutically acceptable salt, [C-4]
      (5) 1-piperazinyl substituted in the 4- position with R₂₂₈-CO-(CH₂)ⱼ- where R₂₂₈ is -X₁, -NR₂₂₉X₁ and 2-furanyl, where R₂₂₉ is -H or C₁-C₃ alkyl, where j is 0 through 3 and X₁ is as defined above, [D]
      (6) 1-piperazinyl substituted in the 4- position with X₂-(CH₂)ⱼ-, where X₂ and j are as defined above, [E]
      (7) 1-piperazinyl substituted in the 4- position with X₁-(CH₂)j-, where X₁ and j are as defined above, [F]
      (8) 4-hydroxy-1-piperidinyl substituted in the 4- position with X₁ as defined above, [G]
      (9) 1-piperazinyl substituted in the 4- position with X₂-NR₂₂₉-CO-(CH₂)ᵢ-, where X₂, R₂₂₉ and i are as defined above; [N]
(D-II) R₁₆ is α-R₁₆₃:β-R₁₆₄ where one of R₁₆₃ and R₁₆₄ is -H and the other is -H, -F, -CH₃ or -OH, and R₁₇ is -CH-(CH₂)ₚ-NR₂₁R₂₁₀, here p is 1 or 2,
   where R₂₁ and R₂₁₀ are as defined above.
(D-III) R₁₆ is α-R₁₆₅:β-R₁₆₆ and R₁₇ is α-R₁₇₅:β-R₁₇₆, where R₁₆₅ is -H, -OH, -F or -CH₃ and R₁₆₆ is -H, -OH, -F, or -CH₃, with the proviso that at least one of R₁₆₅ and R₁₆₆ is -H, where R₁₇₅ is -H, -OH, -CH₃, -CH₂CH₃, C₂-C₇ alkanoyloxy or -O-CO-X₁, where X₁ is as defined above, and where R₁₇₆ is -C(=Z)-(CH₂)n-NR₂₁R₂₁₀, where Z, n, R₂₁ and R₂₁₀ are as defined above;
(D-IV) the 16,17-acetonide of a compound where R₁₆₅ is -OH, R₁₆₆ is -H, R₁₇₅ is -OH and R₁₇₆ is -C(=Z)-(CH₂)ₙ-NR₂₁R₂₁₀, where Z, n, R₂₁ and R₂₁₀ are as defined above;
   and pharmaceutically acceptable salts thereof,
   and hydrates and solvates thereof;
   with the following overall provisos that:
      (I) one of R₁₆₁ or R₁₆₂ is taken together with one of R₁₇₁ or R₁₇₂ to form a second bond between C₁₆ and C₁₇, only when R₁₀ is α-R₁₀₁:β-R₁₀₂, α-R₁₀₃:β-R₁₀₄, α-R₁₀₇:β-R₁₀₈ or α-R₁₀₉:β-R₁₀₁₀,
      (II) R₁₇ is -CH-(CH₂)ₚ-NR₂₁R₂₁₀, only when R₁₀ is α-R₁₀₁:β-R₁₀₂, a-R₁₀₃:β-R₁₀₄, α-R₁₀₇:β-R₁₀₈ or α-R₁₀₉:β-R₁₀₁₀,
      (III) R₅ and R₁₀ taken together are =CH-CH=C(OR₃)-CH=, only when R₁₇ is α-R₁₇₅:β-R₁7₆ or the 16,17-acetonide of a compound where R₁₆ is α-OH:β-H and R₁₇ is α-OH:β-C(=Z)-(CH₂)ₙ-NR₂₁R₂₁₀, and
      (IV) R₅ is α-R₅₇:β-R₅₈, only when R₁₇ is α-R₁₇₅:β-R₁₇₆ or α-OH:β-C-(=Z)-(CH₂)ₙ-NR₂₁R₂₁₀ or the 16,17-acetonide thereof.

More preferred are the C₂₁ aminosteroids of formula XI, especially those which inhibit lipid peroxidation. Most preferred are the 21-[4-(substituted-4-pyrimidinyl)-1-piperazinyl]-steroids, such as U-74006 (21-[4-(2,6-dipyrrolidinyl-4-pyrimidinyl)-1-piperazinyl]-16α-methylpregna-1,4,9(11)-triene- 3,20-dione, and the 21-[4-(substituted-2-pyridinyl)-1-piperazinyl]-steroids, such as U-74500 (21-[4-[5,6-bis(diethylamino)-2-pyridinyl]-1-piperazinyl]-16α-methylpregna-1,4,9(11)-triene-3,20-dione and U-75412 (21-[4-(3-ethylamino-2-pyridinyl)-1-piperazinyl]-16α-methylpregna-1,4,9(11)-triene-3,20-dione, all, when in the unformulated state, preferably as a solid, preferably crystalline, preferably relatively non-hygroscopic and pharmaceutically acceptable salts, such as the methanesulfonate salt of U74006 (U-74006F), the hydrochloride of U-74500 (U-74500A), and the hydrochloride or maleic acid salt of U-75412 (U-75412A and U-75412E, resp.)

The drugs or other medicaments also can be in various forms such as uncharged molecules, components of molecular complexes, pharmacologically acceptable salts such as hydrochloride, hydrobromide, sulfate, laureates, palmatates, phosphate, nitrate, borate, acetate, maleate, tartrate, oleates, and salicylates. For acidic drugs, salts of metals, amines, or organic cations, for example quaternary ammonium can be employed. Furthermore, simple derivatives of the drug such as esters, ethers, and amides which have solubiity characteristics that are suitable for the purpose of the invention may be used. Also, a medicament or drug that is water insoluble can be used in a form that is a water soluble derivative thereof to effectively serve as a solute, and on its release from the device, it is convened by enzymes, hydrolyzed by body pH, or metabolic processes to the original form or to a biologically active form.

The term "pharmaceutically acceptable salt" refers to those salts of the parent compound that do not significantly or adversely affect the pharmaceutical properties (e.g., toxicity, efficacy, etc.) of the parent compound. Pharmaceutically acceptable salts administrable by means of the dispersions or suspensions of this invention include, for example, chloride, iodide, bromide, hydrochloride, acetate, nitrate, stearate, paimoate, phosphate and sulfate salts. It is sometimes desirable to use an appropriate salt form of the drug that increases the water solubility or polar characteristics of the free drug.

Applications disclosed by Choi et al dictate that the free base form of the drug be administered because the acid forms are very water soluble and act as autocatalytic agents which degrade the polymer by acid hydrolysis. With the present invention, on the other hand, use of either the acid form or the free base form of the drug can be undertaken. The acid form is oftentimes more stable than the free base form. When acid forms of drugs are employed, addition of small amounts of a buffer (such as .2% NaH₂PO₄, by weight based on the final formulation) may be desirable.

The present invention is also particularly advantageous for use with water soluble drugs especially when they are suspended or dissolved in the bioerodible polymer of the discontinuous phase. Generally they will not readily diffuse out of the polymer matrix which will thus need to bioerode for delivery of the medicament.

A controlled release drug delivery system of the present invention can be made by a variety of techniques. Basic determinations that should be made include the selection of the bioerodible polymer, selection of the hydrophobic liquid material, and selection of the medicament. Desired system viscosity, giving due attention to the route of administration, should be taken into account. In this connection, use of a plasticizer can be considered.

The choice of the constituents and order of combining the constituents can vary, and certain choices or orders can be more advantageous for certain purposes. For example, when using medicaments that can be unstable at elevated temperatures, it might be desirable to increase flowability of the essentially bioerodible component by adding a plasticizer so that lower temperatures might be employed.

When plasticizers are to be used with the bioerodible polymer, low molecular weight polyethylene glycol (PEG) or polypropylene glycol (PPG) appears particularly appropriate especially in connection with the preferred poly(2,2-dioxy-trans-1,4-cyclohexane dimethylene tetrahydrofuran) polymer. The molecular weight of the PEG or PPG is preferably between about 200 to about 400. The PEG more preferably has a molecular weight of about 300. The low molecular weight PEG or PPG acts as a plasticizer and allows the polymer to be more easily and more uniformly dispersed in the final formulation. Cetyl alcohol is also contemplated as a plasticizer, either alone or more preferably along with polyethylene glycol or glycerin.

The amount of plasticizer employed should be sufficient to soften or make viscous the essentially solid polymer, without adversely interfering with the control release characteristics of the polymer. The total amount of plasticizer will generally be about 0.5% to about 40% by weight of the total weight of the essentially bioerodible component system, more preferably, about 10% to about 20%.

Where it is desired to entrap maximum amounts of medicament in the essentially bioerodible component, that medicament is best mixed with the plasticized or unplasticized essentially bioerodible component before that component is mixed with hydrophobic liquid component. The mixing can desirably involve active agitation, e.g. stirring, and might be enhanced by heating. Certain medicaments can be dissolved in whole or in part by the essentially bioerodible component. Others will be dispersed, with steps such as stirring being undertaken to establish a basically uniform dispersion (e.g., a suspension). Partial dissolution and partial dispersion can also be realized depending upon compatibility.

In most instances it will be desirable at some stage to heat the essentially bioerodible component to render it more flowable. Advantageously, that might be undertaken at the stage of adding the medicament. Heating can be continued or undertaken again or anew when the essentially bioerodible component is mixed with the hydrophobic liquid component. If the initial combination of the bioerodible component and the hydrophobic component occurred without medicament having first been added, heat might advantageously be supplied when the medicament is later added.

Heat can also be advantageously employed to bring viscosities into a desired range. In this regard, high molecular weight polyorthoester constitutents of up to sixty weight per cent have been subjected to temperatures of 140°C to 180°C for 30 minutes to 10 days to reduce molecular weight and provide viscosities of 100 Pa.s. and lower. The temperature and duration of heating to achieve the desired viscosity can depend upon presence or absence of plasticizers, their selection, polymer molecular weight, and concentrations of the polymer.

Whether the essentially bioerodible component is merely dispersed or is suspended (basically uniformly dispersed) in the hydrophobic liquid component can be affected by the order of combining constituents, by employing heat or active mixing, and by use of a plasticizer. Generally, steps to take and maintain the flowability of the essentially bioerodible component at the time of combining that component with the hydrophobic liquid component, e.g., use of plasticizers and application of heat, can render the dispersion more uniform. Active mixing and/or solvent dispersion likewise tends to aid in producing a more uniform dispersion.

When it is preferred that formulations of the present invention be stable over a period of several months or longer, standard or other preservatives can, if desired, be added at any appropriate stage in preparation of the formulations. However, preservative-free packaging in single or limited dose non-reusable containers is also contemplated. Any container used here should be protected from water vapor transport therethrough since water is reactive with the polymer.

When preservatives are to be employed, typical preservatives readily known to those skilled in the art may be determined from any pharmaceutical compounding reference text such as Remingtons Pharmaceutical Sciences. Such preservatives include chlorobutanol, methyl paraben, propyl paraben, and the like, at levels typically ranging from about 0.001 to about 0.5% by weight based on the weight of the final formulation.

Throughout the formulation process, exposure to water or water vapor is desirably minimized or altogether avoided. This is especially true prior to dispersing or suspending the essentially bioerodible component in the hydrophobic liquid component.

Although in certain circumstances, particularly where solvent dispersion is employed, the entire process for preparing a controlled drug delivery system of the present invention might be conducted at room temperature, as mentioned above the use of heat can be advantageous in the manufacturing process, perhaps at the time of addition of each component. The amount of heat will generally be dependent on the polymer selected since different polymers have different melting points. Temperatures employed should also take into account the liquid material selected as the continuous phase. Temperatures used in each step are preferably only slightly above the softening temperatures for the combined ingredients. The final heating step, i.e., once all the components are present, if used, will typically produce a temperature in the range of about 50 to 80°C.

Suitable carriers for injectable or intravenous formulation are well known to persons skilled in the art, e.g., citrate buffer, borate buffer, phosphate buffer and others. Other additives which may be desirably added to parenteral formulations include sodium chloride, sodium sulfite, disodium edetate and benzyl alcohol. Suitable adjuvants for intramuscular formulations are those well known to persons skilled in the art such as polysorbate 80, methyl cellulose, and other demulcents. Other additives desirably added to intramuscular formulations include sodium chloride and sodium bisulfite. Finally, formulations suitable for oral administration will include liquid formulations (solutions, suspension, elixirs, and the like) containing additives and adjuvants well known to persons skilled in the art. Suitable adjuvants may be used as carriers to provide wettability and stability. Other additives, including sodium edetate, flavoring agents and colorants may be employed if desired. Some amounts of acid or base can be used to regulate the release rate of the medicament by controlling the erosion rate through speeding up or slowing down the rate of hydrolysis. Any or all of the foregoing might be employed in certain formulations according to the present invention so long as the basic characteristics of the multi-phase system previously described are maintained.

Conventional encapsulation materials may be used to encapsulate systems of the present invention to provide an encapsulated formulation suitable for oral administration. Alternatively, the present invention may be formulated as a liquid, preferably a viscous liquid, for oral administration.

The following examples are set forth to illustrate the spirit of the present invention. They are not to be construed so as to limit the scope of the invention, as various ways of implementing the present invention will be readily apparent to those skilled in the subject art.

### Example 1

In this example: the essentially biodegradable component which is employed consists essentially of the preferred poly(2,2-dioxy-trans-1,4-cyclohexane dimethylene tetrahydrofuran) polymer, the hydrophobic liquid material is light mineral oil; and the medicament is benzocaine. As a result of the polymerization process, there is acid catalyst, such as para-toluene sulfonic acid, in the polymer. Accordingly, a small amount of a base such as sodium phosphate is employed as a stabilizer to neutralize any residual catalyst. The amounts, in grams, of each ingredient are listed in Table I.

The polymer at a molecular weight of approximately 30,000 and in a solid form, and the other ingredients, are weighed into a small vial containing a magnetic stir bar in a dry environment.

**TABLE I**

| INGREDIENTS | |
|---|---|
| Polymer | 40.00 |
| Light Mineral Oil | 59.20 |
| Sodium Phosphate | 0.50 |
| Benzocaine | 0.30 |

The vial is capped, filled with nitrogen, and the contents are heated for 7 to 10 days at a temperature of about 180°C. The contents are periodically or continuously stirred while the heat is applied. When the heat is removed, the mixture is allowed to cool to room temperature (in about 1 hour) with continuous stirring.

The stirring, during both heating and cooling, is to contribute to the homogeneity of the multi-phase system in which the essentially bioerodible component is suspended as a discontinuous phase in the continuous phase hydrophobic liquid component. It also is to contribute to the uniformity of the distribution of the medicament which in this case may be somewhat soluble in the mineral oil and somewhat soluble in the polymer but is probably mostly suspended, eventually in both phases.

In this regard, at the elevated temperatures the polymer and the oil apparently do not phase separate, so that the medicament can distribute in both the polymer and the oil constituents which are being mixed. At room temperature, the phase separation becomes evident and the medicament is present in both phases.

During the heating process, the molecular weight of the polymer is lowered by as much as an order of magnitude (producing for example a viscosity reduction of from about 30 Pa.s. to about 4 Pa.s.), but not depolymerized to the monomer stage. The depolymerization to the lower molecular weight by heating contributes to maintaining the viscosity of the formulation at about 30 Pa.s, suitable for administration to the eye in drop form. Reductions of the amount of light mineral oil and the increases in polymer content are factors which contribute to increased viscosities. Viscosities are measured on a Brookfield Model LV-cp viscometer with a cp-52 spindle at 0.6 to 3.0 rpm.

Throughout the process of preparing the formulation, precautions are taken to minimize exposure of the polymer to oxygen and water vapor. Heat in the presence of oxygen can cause oxidation which can result in undesirable yellowing (without affecting the erosion rate). Water vapor can cause erosion of the polymer.

Small amounts of the formulation are administered in drop form to the eye of a mammalian organism in need of therapeutic treatment with benzocaine. Controlled release of the benzocaine takes place relatively uniformly over at least a several hour period.

Release profiles may be developed and studied for the formulations, such as by using an in vitro flow-through diffusion apparatus equipped with a peristaltic pump, an ultraviolet detector and a chart recorder. About 10 to 15 microliters of samples of the formulations may be injected through a septum into a sample chamber. Phosphate buffered saline (pH 7.4) may be pumped at a rate, for example, 7.8 ml/hr. Release rates may then be calculated and plotted as a function of time.

### Example 2

In this example, the polymer and the hydrophobic liquid material of Example 1 are employed. However, the molecular weight of the polymer is low (about 2 to about 4 Pa.s.). The medicament employed is interleuken-1 receptor antagonist, a hydrophobic drug which is also sensitive to heat Polymer, light mineral oil, sodium bisulphite and polysorbate 80 are weighed into a vial of the Example 1 type in the following amounts in grams.

**TABLE II**

| INGREDIENTS (initial) | |
|---|---|
| Polymer (low M.W.) | 50.00 |
| Light Mineral Oil | 49.80 |
| Sodium Bisulphite | .10 |
| Polysorbate 80 | .10 |

The sodium bisulphite is present as an antioxidant. The polysorbate 80 is included to aid in dispersing the polymer in the oil.

The vial is capped and the contents are heated for about 30 minutes to about 1 hour to a temperature in the range of about 140°C to about 180°C. The contents are continuously stirred while the heat is applied. After this mixing, the heat is removed and the contents are allowed to cool to room temperature (in about 1 hour) with continuous stirring.

The contents are then reheated to a temperature of about 50°C at which time the medicament, about .1 to about 20 grams, is added, and the entire contents are stirred for 10-30 minutes at which time the heat is removed. Stirring may continue during cooling to room temperature.

As an alternative to cooling, reheating and then cooling again, the temperature of the originally heated mixture can be monitored and the medicament can be added during the initial cooldown stage from about 50°C to room temperature. Continuous stirring takes place during that time.

At the reheat to about 50°C or when that temperature is reached during the initial cooldown, the essentially complete phase separation evident in the desired two phase system at room temperature is not present However, there can be some phase separation. The higher the temperature, the less phase separation, if any, will be present On the other hand, if the medicament cannot tolerate such higher temperature, more phase separation will need to be tolerated at the time of adding the medicament This could inhibit the ability of medicament to actually end up in the polymer matrix. Nevertheless, with hydrophobic medicaments, such as the interleuken-1 receptor antagonist, there is still a tendency for the medicament to partition into the polymer.

In this Example, lower molecular weight of the polymer contributes to maintaining the viscosity at the desired level. The desired viscosity is one suitable for topical opthalmic administration in drop or ribbon form (in a range from about 30 to 55 Pa.s.).

The formulation of this Example is also suitable for injection through an 18 page, or smaller, needle. Small amounts may be injected into the joint of a person suffering from arthritis, in which event the interleuken-1 receptor antagonist, an anti-arthritic agent is controllably released over a prolonged period of time as the essentially bioerodible component is eroded. The formulation of this example may also be administered parenterally to a person suffering from septic shock, for which interleuken-1 receptor antagonist is indicated, in which case it would be controllably released within the body over a prolonged period of time.

### Examples 3-6

In these examples: the essentially biodegradable component which is employed consists essentially of the preferred poly (2,2-dioxy-trans-1,4-cyclohexane diethylene tetrahydrofuran) polymer; the hydrophobic liquid material is silicon oil; and the medicament is proparacaine HCl. Examples 3-4 further include hydrophilic plasticizers, i.e., cetylalcohol and glycerin. Glycerin also acts as a demulcent and as an agent that speeds up the rate of erosion of the biodegradable component. The amount, in grams, of each ingredient is listed in Table III.

**TABLE III**

| INGREDIENTS | Ex. 3 | Ex. 4 | Ex. 5 | Ex. 6 |
|---|---|---|---|---|
| Polymer | 4.6 | 5.5 | 4.0 | 5.5 |
| Silicon Oil 360 | 3.8 | 3.0 | 5.9 | 4.9 |
| Cetyl Alcohol | 1.2 | 1.2 | | |
| Proparacaine HCl | 0.1 | 0.1 | 0.1 | 0.1 |
| Glycerin | 0.3 | 0.2 | | |
| Form of Sample | Drop | Ribbon | Drop | Ribbon |

The polymer at a molecular weight of approximately 17,000 and in an extremely viscous liquid form, along with the plasticizer, i.e., cetyl alcohol and glycerine, if present, are weighed into a small vial containing a magnetic stir bar in a dry environment. The vial is capped, filled with nitrogen, and the contents are heated while continuously stirring for 120 minutes (Examples 3-5) to ten days (Example 6) at 160° to 180°C until the polymer is melted. When the heat is removed, the mixture is allowed to cool to room temperature with continuous stirring. After cooling to room temperature, the weighed amount of hydrophobic liquid material, i.e., silicon oil, is added and again the vial heated for 30 to 120 minutes (Examples 3-5) to ten days (Example 6) at 160° to 180°C while continuously stirring. Then, the medicament is added and the vial is heated for 120 minutes at 70 to 80°C while continuously stirring and again the vial is allowed to cool to room temperature.

Precautions are taken to minimize exposure of the polymer to air by doing all the formulation work in nitrogen purged capped vials and working in a dry box. Completed formulations are placed in unit-dose vials with a syringe and the unit dose tubes are sealed in lamenated aluminum foil bags to prevent water vapor transport through plastic tubes. Sterilization of the formulation is accomplished by Co-60 radiation at a dose of 2.5 mRAD.

### Examples 7-13

In these examples: the essentially biodegradable component which is employed consists essentially of the preferred poly (2,2-dioxy-trans-1,4-cyclohexane diethylene tetrahydrofuran) polymer, and the hydrophobic liquid material is silicon oil. Examples 7 and 11 further include proparacaine HCl as a medicament. Examples 8, 10 and 13 further include polyethylene glycol (PEG 400) as a demulcent and plasticizer. Examples 9, 11 and 12 further include glycerin as a demulcent, plasticizer and agent which moderates the rate of erosion of the biodegradable component The amount in grams, of each ingredient are listed in Table IV.

**TABLE IV**

| INGREDIENTS | Ex. 7 | Ex. 8 | Ex. 9 | Ex. 10 | Ex. 11 | Ex. 12 | Ex. 13 |
|---|---|---|---|---|---|---|---|
| Polymer | 1.0 | 1.0 | 1.0 | 1.0 | 6.0 | 6.0 | 6.0 |
| Silicon Oil 360 | 8.9 | 8.8 | 8.8 | 7.0 | 3.7 | 3.5 | 3.5 |
| PEG-400 | | 0.2 | | 10 | | | 0.5 |
| Proparacaine HCl | 0.1 | | | | 0.1 | | |
| Glycerin | | | 0.2 | | 0.2 | 0.5 | |

The polymer at a molecular weight of approximately 17,000 and in an extremely viscous liquid form, along with the plasticizer, i.e., PEG-400 and glycerin, if present are weighed into a small vial containing a magnetic stir bar in a dry environment. The vial is capped, filled with nitrogen, and the contents are heated for 30 to 120 minutes at 160 to 180°C until the polymer is melted while continuously stirring. When the heat is removed, the mixture is allowed to cool to room temperature with continuous stirring. After cooling to room temperature, the weighed amount of hydrophobic liquid component, i.e., silicon oil, is added and again the vial is heated for 30 minutes to ten days at 160 to 180°C while continuously stirring. Then, the medicament, if present is added and the vial is heated for 120 minutes at 70 to 80°C while continuously stirring and again the vial is allowed to cool to room temperature.

Precautions are taken to minimize exposure of the polymer to air by doing all the formulation work in nitrogen purged capped vials and working in a dry box. Completed formulations are placed in unit-dose vials with a syringe, then closed and sealed in a laminated aluminum overwrap. Sterilization of the formulation is accomplished by Co-60 radiation at a dose of 2.5 mRAD.

### Examples 14-19

In these examples: the essentially biodegradable component which is employed consists essentially of the preferred poly(2,2-dioxy-trans-1,4-cyclohexane diethylene tetrahydrofuran) polymer, the hydrophobic liquid material is silicon oil; and the medicament is proparacaine HCl. Examples 14-15 include no plasticizer. Examples 16-17 include glycerin which acts as a demulcent and a plasticizer and speeds up the rate of erosion of the biodegradable component. Example 18 includes cetyl alcohol as a plasticizer. Example 19 includes polyethylene glycol (PEG-400) as a plasticizer. The amount in grams, of each ingredient are listed in TABLE V.

**TABLE V**

| INGREDIENTS | Ex. 14 | Ex. 15 | Ex. 16 | Ex. 17 | Ex. 18 | Ex. 19 |
|---|---|---|---|---|---|---|
| Polymer | 1.0 | 4.0 | 4.0 | 6.0 | 6.0 | 4.0 |
| Silicon Oil 360 | 8.9 | 5.9 | 5.49 | 3.4 | 3.0 | 5.4 |
| Cetyl Alcohol | | | | | 0.9 | |
| PEG-400 | | | | | | 0.5 |
| Proparacaine HCl | 0.1 | 0.1 | 0.1 | 0.1 | 0.1 | 0.1 |
| Glycerin | | | 0.5 | 0.5 | | |

The polymer at a molecular weight of approximately 17,000 and in an extremely viscous form, along with the hydrophilic plasticizer, if present, are weighed into a small vial containing a magnetic stir bar in a dry environment The vial is capped, filled with nitrogen, and the contents are heated for 30 minutes to ten days at 160 to 180°C until the polymer is melted while continuously stirring. When the heat is removed, the mixture is allowed to cool to room temperature with continuous stirring. After cooling to room temperature, the weighed amount of medicament, is added to the mixture. Then, the mixture is dissolved in 100 grams of ethanol in a sealed flask. The ethanol is stripped off using a Beckman Rotorvapor^{™} under vacuum of 1 mm Hg at a temperature of 30 to 40°C. When all ethanol is removed, the hydrophobic liquid material is added to the mixture and mixed at 30 to 40°C for 2 hours in a flask using a mechanical stirrer.

After mixing, the formulation is placed in unit-dose containers using a syringe, then closed and sealed in laminated aluminum overwrap. Samples are sterilized by Co-60 radiation using a dose of 2.5 mRADS. All work is accomplished in nitrogen capped vials, flasks, or in a dry box.

## Claims

1. A sustained release topical ophthalmic medicament delivery system for administration to the eye, comprising:
a hydrophobic liquid component in a continuous phase;
an essentially bioerodible component which is comprised of a bioerodible polymer and which forms a matrix material for carrying medicament, the bioerodible polymer being a polymer which breaks down by chemical decomposition upon reaction with biological materials;
the essentially bioerodible component being dispersed or suspended as a discontinuous phase in said hydrophobic liquid component;
the relative amounts of said hydrophobic liquid component and said bioerodible component being such that the viscosity of the system is in the range of 1-100 Pa.s and suitable for administration to the eye in ribbon or drop form;
medicament carried in part by the bioerodible component and also carried by or entrapped directly in the continuous phase; and
wherein the essentially bioerodible component acts to hold the formulation, including the medicament, together and, in the topical ophthalmic application, to increase residence time.

2. The delivery system according to claim 1 wherein:
the bioerodible polymer is a polyorthoester.

3. The delivery system according to claim 2 wherein:
the polyorthoester has the formula: 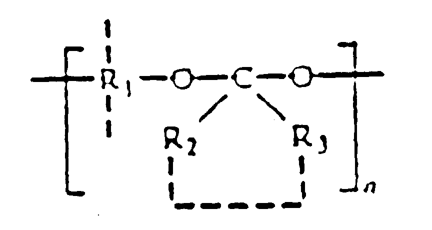 wherein R₁ is a di, tri or tetravalent alkylene, alkenylene, alkyleneoxy, cycloalkylene, cycloalkylene substituted with an alkyl, alkoxy or alkenyl, cycloalkenylene, cycloalkenylene substituted with an alkyl, alkoxy, alkenyloxy, alkylene, alkenylene, alkyleneoxy, alkenyleneoxy, alkylenedioxy, alkenylenedioxy, aryloxy, aralkyleneoxy, aralkenyleneoxy, aralkylene dioxy, aralkenylenedioxy, oxa, or OR₁O with R₁ defined as above; and wherein, a) R₁ is divalent when R₂ and R₃ are alkyl, alkenyl, alkoxy, or alkenyloxy, with at least one of R₂ or R₃ an alkoxy or alkenyloxy; b) R₁ is divalent when R₂ and R₃ are intramolecularly covalently bonded to each other and to the same dioxycarbon atom to form a heterocyclic ring or a heterocyclic ring substituted with an alkyl, alkoxy or alkenyl when R₂ is an alkyleneoxy or alkenyleneoxy and R₃ is an alkyleneoxy, alkenyleneoxy or alkylene; c) R₁ is divalent when R₂ and R₃ are intramolecularly covalently bonded to each other and to the same dioxy carbon atom to form a fused polycyclic ring or a fused polycyclic ring substituted with an alkyl, alkoxy or alkenyl when R₂ is an oxa, alkyleneoxy or alkenyleneoxy and R₃ is aryloxy, aralkyleneoxy, aralkenyleneoxy or aralkylene; d) R₁ is divalent when R₂ or R₃ is an OR₁O bridge between polymer backbones bonded through their dioxycarbon moieties, and the other R₂ or R₃ is an alkyl, alkenyl, alkyloxy, or alkenyloxy; e) R₁ is tri or tetravalent when R₂ and R₃ are covalently bonded to each other and to the same dioxycarbon atom to form a heterocyclic ring or a heterocyclic ring substituted with an alkyl, alkoxy or alkenyl when R₂ is an alkyleneoxy or alkenyleneoxy and R₃ is an alkyleneoxy, or alkylene; f) R₁ is tri or tetravalent when R₂ and R₃ are covalently bonded to each other and to the same dioxy carbon atom to form a fused polycyclic ring or fused polycyclic ring substituted with an alkyl, alkoxy or alkenyl when R₂ is an oxa, alkyleneoxy or alkenyleneoxy and R₃ is aryloxy, aralkyleneoxy, aralkenyleneoxy or aralkylene.

4. The delivery system according to claim 3 wherein:
the polyorthoester is plasticized.

5. The delivery system according to claim 1 wherein:
the bioerodible polymer is poly (2,2-dioxo-trans-1,4-cyclohexane dimethylene tetrahydrofuran).

6. The delivery system according to claim 5 wherein:
the poly (2,2-dioxo-trans-1,4-cyclohexane dimethylene tetrahydrofuran) polymer is plasticized with a plasticizer selected from the group consisting of polyethylene glycol, glycerin, propylene glycol, polypropylene glycol, ethylene glycol, cetyl alcohol and polyvinyl alcohol.

7. The delivery system according to claim 6 wherein;
the essentially bioerodible component is present in an amount within a range of 10% to 60% by weight of the system.

8. The delivery system according to claim 7 wherein:
the plasticizer is present in an amount up to about 40% of the total weight of the essentially bioerodible component.

9. The delivery system according to claim 7 wherein:
the hydrophobic liquid component is selected from the group consisting of mineral oil and silicone oil.

10. The delivery system according to claim 6 wherein:
the viscosity of the system is in the range of 1-55 Pa.s.

11. The delivery system according to claim 1 wherein:
the hydrophobic liquid component is selected from the group consisting of mineral oil and silicone oil.

12. The delivery system according to claim 1 wherein:
the viscosity of the system is in the range of 5-55 Pa.s.

13. The delivery system according to claim 1 wherein:
the essentially bioerodible component is present in an amount within a range of 10% to 60% by weight of the system.

14. The delivery system according to claim 1 wherein:
the medicament is a demulcent.

15. A sustained release medicament delivery system, for administration to the eye, comprising:
a continuous phase liquid component selected from the group consisting of mineral oil and silicone oil;
a bioerodible component selected from the group consisting of plasticized and unplasticized polyorthoesters;
the relative amounts of said liquid component and said bioerodible component being such that the viscosity of the system is in the range of 5-100 Pa.s. and suitable for administration to the eye in ribbon or drop form;
said bioerodible component being dispersed or suspended as a discontinuous phase in said liquid component;
medicament carried in part by the bioerodible component and also carried by or entrapped directly in the continuous phase; and
wherein the essentially bioerodible component acts to hold the formulation, including the medicament, together and, in topical ophthalmic applications, to increase residence time.

16. The sustained release medicament delivery system according to claim 15 wherein:
the bioerodible component is selected from the group consisting of plasticized or unplasticized poly (2,2-dioxo-trans-1,4-cyclohexane dimethylene tetrahydrofuran) polymer.

17. The sustained release delivery system according to claim 15 wherein:
the medicament is a demulcent.

18. A sustained release medicament delivery system, comprising:
a hydrophobic liquid component in a continuous phase; and
an essentially bioerodible component which is comprised of a bioerodible polymer which breaks down by chemical decomposition upon reaction with biological materials;
the essentially bioerodible component being dispersed or suspended as a discontinuous phase in said hydrophobic liquid component;
medicament carried in part by said bioerodible component in said discontinuous phase and also carried by or entrapped directly in the continuous phase; and
wherein the essentially bioerodible component acts to hold the formulation, including the medicament, together.

19. The sustained release medicament delivery system according to claim 15 or 18 wherein:
the system is formulated as an injectable liquid which can be passed through an 18 guage, or smaller, needle at about 20°C.

20. The sustained release delivery system according to claim 15 or 18 wherein:
the system is formulated for oral administration.

21. The sustained release delivery system according to claim 20 wherein the system is formulated in capsule form for oral administration.

22. The system of claim 15 or 18, in the form of a topical ophthalmic composition formulated for dry eye relief and having a viscosity in the range of 1-100 Pa.s.

23. A delivery system according to any preceding claim, wherein the medicament is a water-soluble drug or other water-soluble medicament.

## Patentansprüche

1. Augenmedikament-Abgabesystem mit verzögerter Abgabe zur lokalen Anwendung zur Verabreichung am Auge, umfassend:
eine hydrophobe, flüssige Komponente in einer kontinuierlichen Phase;
eine im wesentlichen bioerodierfähige Komponente, die aus einem bioerodierfähigen Polymer zusammengesetzt ist und die ein Matrixmaterial zur Medikamentenaufnahme bildet, wobei das bioerodierfähige Polymer ein Polymer ist, welches durch chemische Zersetzung bei Reaktion mit biologischen Materialien aufgespalten wird;
wobei die im wesentlichen bioerodierfähige Komponente als eine diskontinuierliche Phase in der hydrophoben, flüssigen Komponente dispergiert oder suspendiert wird;
wobei die relativen Mengen der hydrophoben, flüssigen Komponente und der bioerodierfähigen Komponente derart sind, daß die Viskosität des Systems im Bereich von 1 - 100 Pa.s liegt und für die Verabreichung am Auge in Band- oder Tropfenform geeignet ist;
ein zum Teil durch die bioerodierfähige Komponente getragenes und auch durch die kontinuierliche Phase getragenes oder in dieser direkt eingeschlossenes Medikament; und
wobei die im wesentlichen bioerodierfähige Komponente dazu dient, die Formulierung, einschließlich dem Medikament, zusammen aufzunehmen und um, bei der lokalen Anwendung am Auge, die Verweildauer zu erhöhen.

2. Abgabesystem gemäß Anspruch 1, wobei:
das bioerodierfähige Polymer ein Polyorthoester ist.

3. Abgabesystem gemäß Anspruch 2, wobei:
der Polyorthoester die Formel 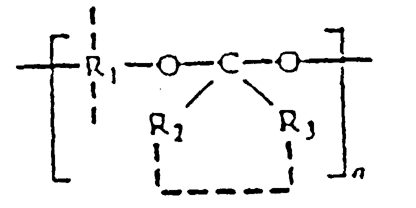 besitzt, worin R ein zwei-, drei- oder vierwertiges Alkylen, Alkenylen, Alkylenoxy, Cycloalkylen, Cycloalkylen, substituiert mit einem Alkyl, Alkoxy oder Alkenyl, Cycloalkenylen, Cycloalkenylen, substituiert mit einem Alkyl, Alkoxy, Alkenyloxy, Alkylen, Alkenylen, Alkylenoxy, Alkenylenoxy, Alkylendioxy, Alkenylendioxy, Aryloxy, Aralkylenoxy, Aralkenylenoxy, Aralkylendioxy, Aralkenylendioxy, Oxa oder OR₁O ist, wobei R₁ wie obenstehend definiert ist; und wobei a) R₁ zweiwertig ist, wenn R₂ und R₃ Alkyl, Alkenyl, Alkoxy oder Alkenyloxy sind, wobei mindestens eines aus R₂ oder R₃ ein Alkoxy oder Alkenyloxy ist; b) R₁ zweiwertig ist, wenn R₂ und R₃ intramolekular kovalent aneinander und an das gleiche Dioxykohlenstoffatom gebunden sind unter Bildung eines heterocyclischen Rings oder eines mit einem Alkyl, Alkoxy oder Alkenyl substituierten heterocyclischen Rings, wenn R₂ ein Alkylenoxy oder Alkenylenoxy ist und R₃ ein Alkylenoxy, Alkenylenoxy oder Alkylen ist; c) R₁ zweiwertig ist, wenn R₂ und R₃ intramolekular kovalent aneinander und an das gleiche Dioxykohlenstoffatom gebunden sind unter Bildung eines verschmolzenen polycyclischen Rings oder eines mit einem Alkyl, Alkoxy oder Alkenyl substituierten verschmolzenen polycyclischen Rings, wenn R₂ ein Oxa, Alkylenoxy oder Alkenylenoxy ist und R₃ Aryloxy, Aralkylenoxy, Aralkenylenoxy oder Aralkylen ist; d) R₁ zweiwertig ist, wenn R₂ oder R₃ eine OR₁O-Brücke zwischen Polymerhauptgerüsten ist, die durch ihre Dioxykohlenstoffreste gebunden sind, und das andere R₂ oder R₃ ein Alkyl, Alkenyl, Alkyloxy oder Alkenyloxy ist; e) R₁ drei- oder vierwertig ist, wenn R₂ und R₃ kovalent aneinander und an das gleiche Dioxykohlenstoffatom gebunden sind unter Bildung eines heterocyclischen Rings oder eines mit einem Alkyl, Alkoxy oder Alkenyl substituierten heterocyclischen Rings, wenn R₂ ein Alkylenoxy oder Alkenylenoxy ist und R₃ ein Alkylenoxy oder Alkylen ist; f) R₁ drei- oder vierwertig ist, wenn R₂ und R₃ kovalent aneinander und an das gleiche Dioxykohlenstoffatom gebunden sind unter Bildung eines verschmolzenen polycyclischen Rings oder eines mit einem Alkyl, Alkoxy oder Alkenyl substituierten verschmolzenen polycyclischen Rings, wenn R₂ ein Oxa, Alkylenoxy oder Alkenylenoxy ist und R₃ Aryloxy, Aralkylenoxy, Aralkenylenoxy oder Aralkylen ist.

4. Abgabesystem gemäß Anspruch 3, wobei:
der Polyorthoester plastifiziert ist.

5. Abgabesystem gemäß Anspruch 1, wobei:
das bioerodierfähige Polymer Poly(2,2-dioxo-trans- 1,4-cyclohexandimethylentetrahydrofuran) ist.

6. Abgabesystem gemäß Anspruch 5, wobei:
das Poly(2,2-dioxo-trans-1,4-cyclohexandimethylentetrahydrofuran)polymer mit einem Weichmacher, ausgewählt aus der Gruppe bestehend aus Polyethylenglykol, Glycerin, Propylenglykol, Polypropylenglykol, Ethylenglykol, Cetylalkohol und Polyvinylalkohol, plastifiziert wird.

7. Abgabesystem gemäß Anspruch 6, wobei:
die im wesentlichen bioerodierfähige Komponente in einer Menge innerhalb eines Bereichs von 10 bis 60 Gew.-% des Systems vorliegt.

8. Abgabesystem gemäß Anspruch 7, wobei:
das Plastifizierungsmittel in einer Menge bis zu etwa 40 % des Gesamtgewichts der im wesentlichen bioerodierfähigen Komponente vorliegt.

9. Abgabesystem gemäß Anspruch 7, wobei:
die hydrophobe, flüssige Komponente ausgewählt ist aus der Gruppe bestehend aus Mineralöl und Siliconöl.

10. Abgabesystem gemäß Anspruch 6, wobei:
die Viskosität des Systems im Bereich von 1 - 55 Pa.s liegt.

11. Abgabesystem gemäß Anspruch 1, wobei:
die hydrophobe, flüssige Komponente ausgewählt ist aus der Gruppe bestehend aus Mineralöl und Siliconöl.

12. Abgabesystem gemäß Anspruch 1, wobei:
die Viskosität des System im Bereich von 5 - 55 Pa.s liegt.

13. Abgabesystem gemäß Anspruch 1, wobei:
die im wesentlichen bioerodierfähige Komponente in einer Menge innerhalb eines Bereichs von 10 bis 60 Gew.-% des Systems vorliegt.

14. Abgabesystem gemäß Anspruch 1, wobei:
das Medikament ein Demulcens ist.

15. Medikament-Abgabesystem mit verzögerter Freisetzung zur Verabreichung am Auge, umfassend:
eine flüssige Komponente einer kontinuierlichen Phase, ausgewählt aus der Gruppe bestehend aus Mineralöl und Siliconöl;
eine bioerodierfähige Komponente, ausgewählt aus der Gruppe bestehend aus plastifizierten und nichtplastifizierten Polyorthoestern;
wobei die relativen Mengen der flüssigen Komponente und der bioerodierfähigen Komponente derart sind, daß die Viskosität des Systems im Bereich von 5 - 100 Pa.s liegt und sich für die Verabreichung am Auge in Band- oder Tropfenform eignet;
wobei die bioerodierfähige Komponente als diskontinuierliche Phase in der flüssigen Komponente dispergiert oder suspendiert ist;
wobei das Medikament zum Teil durch die bioerodierfähige Komponente getragen und auch durch die kontinuierliche Phase getragen oder in dieser direkt eingeschlossen wird:
wobei die im wesentlichen bioerodierfähige Komponente dazu dient, die Formulierung, einschließlich dem Medikament, zusammen aufzunehmen und um, bei der lokalen Anwendung am Auge, die Verweildauer zu erhöhen.

16. Medikament-Abgabesystem mit verzögerter Freisetzung gemäß Anspruch 15, wobei:
die bioerodierfähige Komponente ausgewählt ist aus der Gruppe bestehend aus plastifiziertem oder nichtplastifiziertem Poly(2,2-dioxo-trans-1,4-cyclohexandimethylentetrahydrofuran)polymer.

17. Abgabesystem mit verzögerter Freisetzung gemäß Anspruch 15, wobei:
das Medikament ein Demulcens ist.

18. Medikament-Abgabesystem mit verzögerter Freisetzung, umfassend:
eine hydrophobe, flüssige Komponente in einer kontinuierlichen Phase; und eine im wesentlichen bioerodierfähige Komponente, die aus einem bioerodierfähigen Polymer zusammengesetzt ist, welches durch chemische Zersetzung bei Reaktion mit biologischen Materialien aufgespalten wird;
wobei die im wesentlichen bioerodierfähige Komponente als eine diskontinuierliche Phase in der hydrophoben, flüssigen Komponente dispergiert oder suspendiert wird;
ein zum Teil durch die bioerodierfähige Komponente in der diskontinuierlichen Phase getragenes und auch durch die kontinuierliche Phase getragenes oder in dieser direkt eingeschlossenes Medikament; und
wobei die im wesentlichen bioerodierfähige Komponente dazu dient, die Formulierung, einschließlich dem Medikament, zusammen aufzunehmen.

19. Medikament-Abgabesystem mit verzögerter Freisetzung gemäß Anspruch 15 oder 18, wobei:
das System als eine injizierbare Flüssigkeit formuliert ist, welche durch eine Nadel mit einem Kaliber von 18 oder kleiner bei etwa 20°C geleitet wird.

20. Medikament-Abgabesystem mit verzögerter Freisetzung gemäß Anspruch 15 oder 18, wobei:
das System für die orale Verabreichung formuliert ist.

21. Abgabesystem mit verzögerter Freisetzung gemäß Anspruch 20, wobei das System in Kapselform zur oralen Verabreichung formuliert ist.

22. System nach Anspruch 15 oder 18 in der Form einer lokal an den Augen anzuwendenden Zusammensetzung, formuliert für eine Linderung bzw. Beruhigung von trockenen Augen und mit einer Viskosität im Bereich von 1 - 100 Pa.s.

23. Abgabesystem gemäß mindestens einem der vorhergehenden Ansprüche, wobei das Medikament ein wasserlösliches Arzneimittel oder ein anderes wasserlösliches Medikament ist.

## Revendications

1. Un système de délivrance de médicament ophtalmique topique à libération prolongée pour l'administration à l'oeil, comprenant:
un constituant liquide hydrophobe dans une phase continue ;
un constituant essentiellement bioérodable qui est composé d'un polymère bioérodable et qui forme un matériau de matrice pour transporter un médicament, le polymère bioérodable étant un polymère qui se dissocie par décomposition chimique suite à une réaction avec des matières biologiques ;
le constituant essentiellement bioérodable étant dispersé ou en suspension sous la forme d'une phase discontinue dans ledit constituant liquide hydrophobe ;
les quantités relatives dudit constituant liquide hydrophobe et dudit constituant bioérodable étant telles que la viscosité du système est dans la gamme de 1 à 100 Pa.s et convient pour l'administration à l'oeil sous forme de ruban ou de goutte ;
un médicament transporté en partie par ledit constituant bioérodable et aussi transporté par ou piégé directement dans la phase continue ; et
dans lequel le constituant essentiellement bioérodable sert à maintenir la formulation, y compris le médicament, en un ensemble et, lors de l'application ophtalmique topique, à accroître le temps de séjour.

2. Le système de délivrance selon la revendication 1, dans lequel : le polymère bioérodable est un polyorthoester.

3. Le système de délivrance selon la revendication 2, dans lequel : le polyorthoester a la formule : 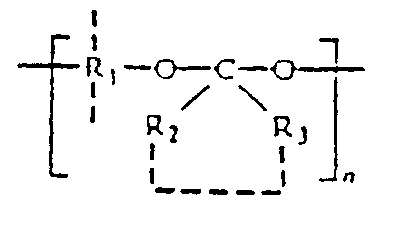 dans laquelle R₁ est un groupe di, tri ou tétravalent alkylène, alcénylène, alkylèneoxy, cycloalkylène, cycloalkylène substitué par un groupe alkyle, alcoxy ou alcényle, cycloalcénylène, cycloalcénylène substitué par un groupe alkyle, alcoxy, alcényloxy, alkylène, alcénylène, alkylèneoxy, alcénylèneoxy, alkylènedioxy, alcénylènedioxy, aryloxy, aralkylèneoxy, aralcénylèneoxy, aralkylènedioxy, aralcénylènedioxy, oxa ou OR₁O avec R₁ défini comme ci-dessus ; et dans laquelle a) R₁ est divalent lorsque R₂ et R₃ sont un groupe alkyle, alcényle, alcoxy ou alcényloxy, au moins l'un parmi R₂ et R₃ étant un groupe alcoxy ou alcényloxy ; b) R₁ est divalent lorsque R₂ et R₃ sont liés intramoléculairement de façon covalente l'un à l'autre et au même atome de dioxycarbone pour former un noyau hétérocyclique ou un noyau hétérocyclique substitué par un groupe alkyle, alcoxy ou alcényle lorsque R₂ est un groupe alkylèneoxy ou alcénylèneoxy et R₃ est un groupe alkylèneoxy, alcénylèneoxy ou alkylène ; c) R₁ est divalent lorsque R₂ et R₃ sont liés intramoléculairement de façon covalente l'un à l'autre et au même atome de dioxycarbone pour former un noyau polycyclique condensé ou un noyau polycyclique condensé substitué par un groupe alkyle, alcoxy ou alcényle lorsque R₂ est un groupe oxa, alkylèneoxy ou alcénylèneoxy et R₃ est un groupe aryloxy, aralkylèneoxy, aralcénylèneoxy ou aralkylène ; d) R₁ est divalent lorsque R₂ ou R₃ est un pont OR₁O entre des squelettes polymères liés par leurs parties dioxycarbonées, et l'autre R₂ ou R₃ est un groupe alkyle, alcényle, alkyloxy ou alcényloxy ; e) R₁ est tri ou tétravalent lorsque R₂ et R₃ sont liés de façon covalente l'un à l'autre et au même atome de dioxycarbone pour former un noyau hétérocyclique ou un noyau hétérocyclique substitué par un groupe alkyle, alcoxy ou alcényle lorsque R₂ est un groupe alkylèneoxy ou alcénylèneoxy et R₃ est un groupe alkylèneoxy ou alkylène ; f) R₁ est tri ou tétravalent lorsque R₂ et R₃ sont liés de façon covalente l'un à l'autre et au même atome de dioxycarbone pour former un noyau polycyclique condensé ou un noyau polycyclique condensé substitué par un groupe alkyle, alcoxy ou alcényle lorsque R₂ est un groupe oxa, alkylèneoxy ou alcénylèneoxy et R₃ est un groupe aryloxy, aralkylèneoxy, aralcénylèneoxy ou aralkylène.

4. Le système de délivrance selon la revendication 3, dans lequel : le polyorthoester est plastifié.

5. Le système de délivrance selon la revendication 1, dans lequel : le polymère bioérodable est un poly(2,2-dioxo-trans-1,4-cyclohexane diméthylène tétrahydrofuranne).

6. Le système de délivrance selon la revendication 5, dans lequel : le polymère poly(2,2-dioxo-trans-1,4-cyclohexane diméthylène tétrahydrofuranne) est plastifié avec un plastifiant choisi dans le groupe constitué par un polyéthylèneglycol, la glycérine, le propylèneglycol, un polypropylèneglycol, l'éthylèneglycol, l'alcool cétylique et un alcool polyvinylique.

7. Le système de délivrance selon la revendication 6, dans lequel : le constituant essentiellement bioérodable est présent en une proportion dans la gamme de 10 % à 60 % en poids par rapport au système.

8. Le système de délivrance selon la revendication 7, dans lequel : le plastifiant est présent en une proportion allant jusqu'à environ 40 % du poids total du constituant essentiellement bioérodable.

9. Le système de délivrance selon la revendication 7, dans lequel : le constituant liquide hydrophobe est choisi dans le groupe constitué par une huile minérale et une huile de silicone.

10. Le système de délivrance selon la revendication 6, dans lequel : la viscosité du système est dans la gamme de 1 à 55 Pa.s.

11. Le système de délivrance selon la revendication 1, dans lequel : le constituant liquide hydrophobe est choisi dans le groupe constitué par une huile minérale et une huile de silicone.

12. Le système de délivrance selon la revendication 1, dans lequel : la viscosité du système est dans la gamme de 5 à 55 Pa.s.

13. Le système de délivrance selon la revendication 1, dans lequel : le constituant essentiellement bioérodable est présent en une proportion dans la gamme de 10 % à 60 % en poids par rapport au système.

14. Le système de délivrance selon la revendication 1, dans lequel : le médicament est un émollient.

15. Un système de délivrance de médicament à libération prolongée, à administrer à l'oeil, comprenant :
un constituant liquide à phase continue choisi dans le groupe constitué par une huile minérale et une huile de silicone ;
un constituant bioérodable choisi dans le groupe constitué par les polyorthoesters plastifiés et non plastifiés ;
les quantités relatives dudit constituant liquide et dudit constituant bioérodable étant telles que la viscosité du système est dans la gamine de 5 à 100 Pa.s et convient pour l'administration à l'oeil sous forme de ruban ou de goutte ;
ledit constituant bioérodable étant dispersé ou en suspension sous la forme d'une phase discontinue dans ledit constituant liquide ;
un médicament transporté en partie par ledit constituant bioérodable et aussi transporté par ou piégé directement dans la phase continue ; et
dans lequel le constituant essentiellement bioérodable sert à maintenir la formulation, y compris le médicament, en un ensemble, lors d'applications ophtalmiques topiques, à accroître le temps de séjour.

16. Le système de délivrance de médicament à libération prolongée selon la revendication 15, dans lequel : le constituant bioérodable est choisi dans le groupe constitué par un polymère poly(2,2-dioxo-trans-1,4-cyclohexane diméthylène tétrahydrofuranne) plastifié ou non plastifié.

17. Le système de délivrance à libération prolongée selon la revendication 15, dans lequel : le médicament est un émollient.

18. Un système de délivrance de médicament à libération prolongée, comprenant :
un constituant liquide hydrophobe dans une phase continue ; et
un constituant essentiellement bioérodable qui est composé d'un polymère bioérodable qui se dissocie par décomposition chimique suite à une réaction avec des matières biologiques ;
le constituant essentiellement bioérodable étant dispersé ou en suspension sous la forme d'une phase discontinue dans ledit constituant liquide hydrophobe ;
un médicament transporté en partie par ledit constituant bioérodable dans ladite phase discontinue et aussi transporté par ou piégé directement dans la phase continue ; et
dans lequel le constituant essentiellement bioérodable sert à maintenir la formulation, y compris le médicament, en un ensemble.

19. Le système de délivrance de médicament à libération prolongée selon la revendication 15 ou 18, dans lequel : le système est formulé sous la forme d'un liquide injectable qu'on peut faire passer à travers une aiguille de calibre 18, ou inférieur, à environ 20 °C.

20. Le système de délivrance à libération prolongée selon la revendication 15 ou 18, dans lequel : le système est formulé pour l'administration orale.

21. Le système de délivrance à libération prolongée selon la revendication 20, le système étant formulé sous forme de capsule pour l'administration orale.

22. Le système de la revendication 15 ou 18, sous la forme d'une composition ophtalmique topique formulée pour remédier au syndrome de l'oeil sec et ayant une viscosité dans la gamine de 1 à 100 Pa.s.

23. Un système de délivrance selon l'une quelconque des revendications précédentes, dans lequel le médicament est un médicament hydrosoluble ou un autre médicament hydrosoluble.
